# EUROPÄISCHE PATENTSCHRIFT

(11) **EP 4 100 387 B1**
(45) Veröffentlichungstag und Bekanntmachung des Hinweises auf die Patenterteilung: **31.05.2023**
(21) Anmeldenummer: 21791407.6
(22) Anmeldetag: 19.10.2021
(51) Int. Cl.: C07C 51/06, C07C 67/20, C07C 231/06, C07C 231/12, C07C 303/24

(54) **OPTIMIERTES VERFAHREN ZUR HERSTELLUNG VON METHACRYLSÄURE (MAS) UND/ODER ALKYLMETHACRYLAT DURCH REDUZIERUNG STÖRENDER NEBENPRODUKTE**
OPTIMIZED METHOD FOR THE PREPARATION OF METHACRYLIC ACID AND/OR ALKYL METHACRYLATE BY REDUCING INTERFERING BYPRODUCTS
PROCÉDÉ OPTIMISÉ DE FABRICATION D'ACIDE MÉTHACRYLIQUE ET/OU DE MÉTHACRYLATE D'ALKYLE PAR RÉDUCTION DES SOUS-PRODUITS INTERFÉRENTS

(30) Priorität: 23.10.2020 EP 20203724
(43) Veröffentlichungstag der Anmeldung: 14.12.2022
(73) Patentinhaber: Röhm GmbH, 64295 Darmstadt (DE)
(72) Erfinder: KRILL, Steffen, 64367 Mühltal (DE); KLASOVSKY, Florian, 45721 Haltern am See (DE); KÖNIG, Daniel, Helmut, 70499 Stuttgart (DE); WINGS, Patrick, 50935 Köln (DE)
(74) Vertreter: Röhm Patent Association
(86) Internationale Anmeldenummer: PCT/EP2021/078866
(87) Internationale Veröffentlichungsnummer: WO 2022/084274

(56) Entgegenhaltungen:
- EP-A1- 1 359 137
- WO-A1-2014/051971
- DE-A1-102004 006 826
- US-A- 4 529 816

## Beschreibung

Die vorliegende Erfindung betrifft ein verbessertes Verfahren zur Herstellung von Methacrylsäure (MAS) und / oder Alkylmethacrylaten, insbesondere Methylmethacrylat (MMA), umfassend die Umsetzung von Aceton und Blausäure in Gegenwart eines basischen Katalysators in einer ersten Reaktionsstufe, wobei eine erste Reaktionsmischung enthaltend Acetoncyanhydrin (ACH) erhalten wird, die Aufarbeitung der ersten Reaktionsmischung enthaltend Acetoncyanhydrin (ACH), die Umsetzung von Acetoncyanhydrin (ACH) und Schwefelsäure in einer zweiten Reaktionsstufe (Amidierung) und die Erhitzung der zweiten Reaktionsmischung in einer dritten Reaktionsstufe (Konvertierung), wobei Methacrylamid (MASA) erhalten wird, sowie die anschließende Hydrolyse bzw. Veresterung von Methacrylamid (MASA) mit Wasser bzw. mit Alkohol und Wasser, bevorzugt Methanol und Wasser, in einer vierten Reaktionsstufe unter Bildung von Methacrylsäure bzw. Alkylmethacrylat, wobei die eingesetzte Schwefelsäure eine Konzentration im Bereich von 98,0 Gew.-% bis 100,0 Gew.-% aufweist.

Insbesondere betrifft die vorliegende Erfindung ein optimiertes Verfahren zur Herstellung von Methacrylsäure und / oder Alkylmethacrylat, umfassend die spezifische Einstellung und Kontrolle der Qualität der Zwischenprodukte und Produkte, insbesondere MASA und MMA, wobei die Bildung störender Nebenprodukte, insbesondere Methacrylnitril (MAN), Aceton, Isobuttersäuremethylester (IBSME) und Propionsäuremethylester (Methylpropionat, MP) sowie Diacetyl (Di-Ac, Butan-2,3-dion), in den Vorstufen und Zwischenprodukten reduziert wird, und die Ausbeute an Zwischenprodukten und Produkten verbessert wird.

### Stand der Technik

Methylmethacrylat (MMA) wird in großen Mengen zur Herstellung von Polymeren und Copolymeren mit anderen polymerisierbaren Verbindungen eingesetzt. Weiterhin ist Methylmethacrylat ein wichtiger Baustein für verschiedene Spezialester, basierend auf Methacrylsäure (MAS), die durch Umesterung vom MMA mit dem entsprechenden Alkohol hergestellt werden können oder durch Kondensation von Methylacrylsäure und einem Alkohol bzw. Aminoalkohol zugänglich sind. Hieraus ergibt sich ein großes Interesse an möglichst einfachen, wirtschaftlichen und umweltschonenden Herstellungsverfahren für diesen Ausgangsstoff.

Die Herstellung von Methacrylamid durch die Amidierung von Acetoncyanhydrin (ACH) ist ein weithin angewandtes Verfahren. Beispielsweise stellt eine derartige Amidierung, welche im Stand der Technik auch als ACH-Hydrolyse bezeichnet wird, einen wichtigen Zwischenschritt bei der Herstellung von Methylmethacrylat gemäß dem sogenannten ACH-Sulfo-Verfahren dar, wobei große Mengen an Schwefelsäure im Prozess eingesetzt werden. Die Herstellung von Methacrylsäure (MAS) und Methylmethacrylat (MMA) nach dem ACH-Sulfo-Verfahren ist allgemein bekannt und im Stand der Technik beschreiben, z.B. WO 2008/068064, WO 2013/143812, EP 0 226 724. Hierbei wird zunächst in einem ersten Schritt Acetoncyanhydrin (ACH) durch Umsetzung von Blausäure und Aceton hergestellt, welches anschließend zum Methacrylamid (MASA) umgesetzt wird. Diese Schritte sind u.a. in US 7,253,307, EP 1 666 451 oder EP 2007 059092 dargestellt. Die Umsetzung von ACH zu MASA (Amidierung) wird üblicherweise durch eine Reaktion zwischen höchstkonzentrierter Schwefelsäure, insbesondere Oleum, und ACH bewirkt. Die Umsetzung ist exotherm, so dass bevorzugt die Reaktionswärme schnell aus dem System abgeführt wird.

Die Umsetzung zu MASA verläuft typischerweise in zwei Prozessschritten. Zunächst wird im Amidierungsschritt eine schwefelsaure, im Wesentlichen wasserfreie Lösung, enthaltend hauptsächlich alpha-Hydroxyisobuttersäureamid (alpha-Hydroxybutyramid) (HIBA), dessen Sulfatester alpha-Sulfoxyisobuttersäureamid (SIBA) und Methacrylamid (MASA) (bzw. salzartig protoniert in Form der jeweiligen Hydrogensulfate), erhalten. Im anschließenden Schritt, der so genannten Konvertierung, wird diese Lösung typischerweise bei hohen Temperaturen zwischen 130 °C bis 200 °C und meist kurzen Verweilzeiten, z.B. von etwa 20 min oder weniger, unter β-Eliminierung von Wasser bzw. Schwefelsäure zu Methacrylamid (MASA) umgesetzt. Typischerweise liegt nach der Konvertierung das Hauptprodukt MASA mit einer Konzentration in der Lösung von etwa 30 Gew.-% bis 40 Gew.-% (je nach eingesetztem Schwefelsäureüberschuss) vor.

Die Schritte der Amidierung und der Konvertierung unterscheiden sich in der Regel prozesstechnisch wesentlich in der Verweilzeit und auch im eingesetzten Temperaturniveau. Im Hinblick auf die chemische Reaktion wird die Amidierung typischerweise kürzer als die Konvertierung und typischerweise bei niedrigeren Temperaturen als die Konvertierung durchgeführt.

Das Dokument US-B 4,529,816 beschreibt ein Verfahren zur Herstellung von Alkylmethacrylaten nach dem ACH-Sulfo-Verfahren, wobei die Amidierung bei Temperaturen um 100 °C mit weit überstöchiometrischen Mengen an Schwefelsäure (molares Verhältnis von ACH : H₂SO₄ von etwa 1:1,3 bis 1:1,8) durchgeführt wird. Es wird darauf hingewiesen, dass die eingesetzte Schwefelsäure ausreichend SO₃ enthalten sollte, um mindestens eine Säurestärke von 98%, bevorzugt mindestens 99,3% zu gewährleisten. Insbesondere soll rauchende Schwefelsäure (Oleum) eingesetzt werden, welche bevorzugt 101 % Schwefelsäure und somit freies SO₃ aufweist. Im nachfolgenden Schritt der Veresterung wird die Reaktionsmischung mit einem Überschuss Wasser und Alkohol bei 100 bis 150 °C behandelt. US-B 4,529,816 beschreibt, dass das Nebenprodukt alpha-Hydroxyisobuttersäuremethylester (HIBSM) nach der Veresterung vom Methylmethacrylat-Produkt abgetrennt und in das Verfahren zurückgeführt wird. In ähnlicher Weise werden in dem Verfahren nach US 5,393,918 Nebenprodukte, unter anderem alpha-Hydroxyisobuttersäuremethylester (HIBSM) isoliert, dehydratisiert und in die Reaktion zurückgeführt. Es wird beschreiben, dass die Konzentration der Schwefelsäure bei der Umsetzung von ACH nicht entscheidend ist und im Bereich von 95 bis 100 % gewählt wird.

Das Dokument EP 1 359 137 A1 beschreibt ein Verfahren zur Herstellung von Methacrylsäure und Estern davon, umfassend die Reaktion von Acetocyanohydrin mit Schwefelsäure, wobei die Konzentration der Schwefelsäure bevorzugt höher als 95 % ist.

Das Dokument DE 38 28 253 A1 beschreibt ein Verfahren zur Rückführung verbrauchter Schwefelsäure bei der Herstellung von Methacrylsäureestern nach dem ACH-Sulfo-Verfahren, wobei die verbrauchte Säure nach der Veresterung aufkonzentriert, mit frischer Säure gemischt und zurückgeführt wird. DE 38 28 253 A1 beschreibt allgemein eine Säurestärke von 96 bis 101 % bei der Umsetzung des Acetoncyanhydrins mit Schwefelsäure.

Das Dokument DE 1 618 721 beschreibt die Umsetzung von Acetoncyanhydrin (ACH) mit Schwefelsäure in zwei Stufen mit unterschiedlichem Verhältnis von Schwefelsäure zu ACH, wodurch die Viskosität der Reaktionsmischung geregelt werden soll. In dem in EP 0 226 724 beschriebenen Verfahren wird die Umsetzung in Gegenwart eines Alkan-Lösungsmittels durchgeführt, um die Viskosität der Reaktionsmischung und Reaktionsenthalpie zu regeln. Das Dokument CH 239749 beschreibt ein Verfahren zur Herstellung von Methacrylamid durch Einwirkung von Schwefelsäure auf Acetoncyanhydrin bei Temperaturen von 110-130 °C und 115-160 °C, wobei beispielsweise 100 %ige Schwefelsäure eingesetzt wird. Alternativ zur Umsetzung von schwefelsauren MASA-Lösungen mit Alkoholen und Wasser zu Alkylmethacrylaten kann die nach der Konvertierung erhaltene schwefelsaure MASA-Lösung auch mit Wasser zur Methacrylsäure umgesetzt werden.

US-B 4,748,268 beschreibt ein Verfahren zur Veresterung von Methacrylsäure mit einem C₁-C₄ Alkohol in Gegenwart einer hochsiedenden organischen Flüssigkeit in einem Plug-Flow-Reaktor, bei dem das Reaktionsgemisch kontinuierlich fraktioniert wird, wobei der Destillatstrom einen höheren Anteil an Methacrylsäureester aufweist und der Sumpfstrom zum überwiegenden Teil in den Plug-Flow-Reaktor zurückgeführt wird.

Als Nebenprodukte bei der Amidierung und Konvertierung werden unter anderem Kohlenstoffmonoxid, Aceton, Sulfonierungsprodukte des Acetons und Cyclokondensationsprodukte von Aceton mit diversen Intermediaten gebildet. Diese genannten Nebenprodukte können meist relativ effektiv vom Alkylmethacrylat-Produkt abgetrennt werden. Darüber hinaus bilden sich allerdings auch - in Abhängigkeit der Reaktionsbedingungen - andere Nebenprodukte, deren Abtrennung vom Alkylmethacrylat, insbesondere vom Methylmethacrylat-Produkt, schwierig beziehungsweise mit erheblichem Trennaufwand verbunden ist. Beispielsweise gestaltet sich die Trennung aufgrund der Azeotrop-Siedepunkte und der Siedepunkte der spezifischen Verbindungen schwierig. Diese störenden Nebenprodukte sind insbesondere Methacrylnitril (MAN), Aceton, Isobuttersäuremethylester (IBSME) und Propionsäuremethylester (Methylpropionat, MP) sowie Diacetyl (Di-Ac, Butan-2,3-dion). Einige dieser störenden Nebenprodukte sind maßgeblich für eine erhöhte Farbzahl im Alkylmethacrylat-Endprodukt, insbesondere MMA, verantwortlich. Insbesondere die Diacetylabtrennung muss effektiv und bevorzugt vollständig erfolgen, weil zum einen nicht unerhebliche Mengen während der Reaktionen gebildet werden und zum anderen der Stoff maßgeblich zur Farbzahl im finalen Endprodukt MMA beiträgt, sofern er im Produkt in ppm-Konzentrationen nachzuweisen ist.

Diese und andere störende niedermolekularen Nebenprodukte können zudem Probleme bei der weiteren Polymerisation und Verarbeitung der Polymere machen, z.B. durch Ausgasen bei der Extrusion oder im Spitzguss. Störende Nebenprodukte mit einer Doppelbindung werden neben dem Alkylmethacrylat ins Polymerprodukt einpolymerisiert und beeinträchtigen die Eigenschaften der Polymere, z.B. die Transparenz, negativ. Um spezifikationsgerechtes Alkylmethacrylat-Endprodukt, insbesondere MMA, zu erhalten, müssen diese Nebenprodukte, wie MAN, IBSME und/oder MP, in den Reaktionsschritten reduziert oder in der Aufarbeitung entfernt werden.

Methacrylnitril (MAN) bildet sich typischerweise als Nebenprodukt während der Amidierungsreaktion und bei der SIBA-Eliminierung zu MASA in der Konvertierung aus Acetoncyanhydrin (ACH) unter Eliminierung von Wasser.

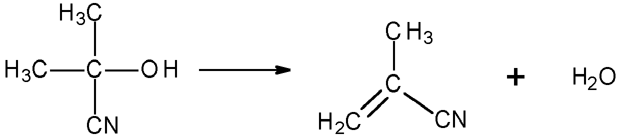

Methacrylnitril (MAN) bildet sowohl mit Methanol (MeOH) als auch mit anderen im System vorhandenen Stoffen Azeotrope bzw. ist nur mit nicht unerheblichem Aufwand von MMA-Azeotropen (z.B. die Azeotrope mit Wasser und Methanol) aufgrund ähnlicher Siedepunkte vom Produkt abzutrennen. Weiterhin verhält sich MAN bezüglich der Mischungseigenschaften und seiner Polarität ähnlich wie MMA und ist deshalb in extraktiven Schritten und Phasentrennungsapparaten nur schwierig in diesen Operationen vom MMA trennbar. Eine vollständige Abtrennung von MAN ist mit vernünftigem Aufwand in der Regel nicht möglich und bedarf besonderer Maßnahmen.

Wünschenswert in diesem Zusammenhang ist insbesondere, Möglichkeiten zu finden, die auch eine Beeinflussung der Bildung, konkret eine Reduzierung der Bildung, in den verschiedenen Reaktionsschritten erlaubt.

Bei der Amidierung werden als gewünschte Hauptprodukte der Umsetzung Sulfoxyisobuttersäureamid-Hydrogensulfat (SIBA*H₂SO₄) und Methacrylamid-Hydrogensulfat (MASA*H₂SO₄) als Lösung in überschüssiger Schwefelsäure erhalten. Des Weiteren ist typischerweise in der Amidierungslösung noch alpha-Hydroxyisobuttersäureamid-Hydrogensulfat (HIBA·H₂SO₄) enthalten, beispielsweise mit einer Ausbeute bezüglich ACH von < 5%. Bei annähernd vollständigem Umsatz von ACH kann im Amidierungsschritt eine Ausbeute an den oben beschriebenen Intermediaten von typischerweise etwa 94 % bis 96 % erhalten werden. In diesem Schritt werden oftmals in erheblichen Mengen die oben beschriebenen Nebenprodukte gebildet.

Aus der Ammoniumhydrogensulfat- und Schwefelsäure-haltigen Prozesssäure, welche oftmals in einer Schwefelsäurekontaktanlage regeneriert wird, scheiden sich zudem teerartige, feste Kondensationsprodukte ab, die eine Förderung der Prozesssäure behindern und unter erheblichem Aufwand beseitigt und entsorgt werden müssen.

Es ist außerdem bekannt, dass Hydroxyisobuttersäure ausgehend von Acetoncyanhydrin (ACH) durch Verseifung der Nitrilfunktion in Gegenwart von Mineralsäuren hergestellt werden kann. Im Stand der Technik werden Verfahren beschrieben, bei denen ACH in Gegenwart von Wasser amidiert und hydrolysiert wird, wobei die Hydroxy-Funktion im Molekülverbund zumindest in den ersten Schritten der Umsetzung erhalten bleibt, z.B. WO 2005/077878, JP H04 193845 A, JP S57 131736. Beispielsweise wird in der japanischen Patentanmeldung JP S6361932B2 beschrieben, dass ACH in einem zweistufigen Prozess zur Hydroxyisobuttersäure verseift wird, wobei ACH zunächst in Gegenwart von 0,2 bis 1,0 mol Wasser und 0,5 bis 2 Äquivalenten Schwefelsäure umgesetzt wird, wobei die entsprechenden Amidsalze gebildet werden. Diese Vorschläge zu einer alternativen Amidierung in Gegenwart von Wasser führen, je nachdem, ob in Gegenwart von Methanol oder ohne Methanol durchgeführt, entweder zur Entstehung von Hydroxyisobuttersäuremethylester (HIBSM) oder zur Entstehung von 2-Hydroxyisobuttersäure (HIBS). Beispielsweise erfolgt gemäß JP S57 131736 die Umsetzung von ACH mit 0,8 bis 1,25 Äquivalenten Schwefelsäure in Gegenwart von weniger als 0,8 Äquivalenten Wasser unterhalb 60 °C und anschließend die Umsetzung mit mehr als 1,2 Äquivalenten Methanol zum HIBSM bei Temperaturen von größer als 55 °C.

Das Verfahren zur Herstellung von MAS wird im Wesentlichen analog zur Herstellung von MMA durchgeführt. Bei der MAS-Herstellung und der MMA-Herstellung sind oftmals Amidierung und Konvertierung im Wesentlichen ähnlich oder sogar identisch, da insbesondere eine möglichst hohe MASA-Ausbeute aus ACH angestrebt wird. Wenn die MASA-haltige dritte Reaktionsmischung ohne Alkohol nur mit Wasser umgesetzt wird, führt die Reaktion typischerweise zu MAS-Rohgemischen als vierte Reaktionsmischung, während in Gegenwart von Wasser und Alkohol (z.B. Methanol) die Reaktion zu Alkylmethacrylaten (z.B. MMA) in einer vierten Reaktionsmischung führt. Dem Fachmann ist in diesem Zusammenhang bekannt, dass für die selektive Herstellung von MAS höhere Stoffmengenanteile an Wasser zur Verfügung gestellt werden müssen, als dies bei der MMA-Herstellung der Fall ist.

Keines der Verfahren im Stand der Technik beschreibt eine Möglichkeit zur Kontrolle und Reduzierung von störenden Nebenprodukten, insbesondere von Methacrylnitril (MAN) und Aceton, welche die Eigenschaften des Alkylmethacrylats und der Alkylmethacrylat-Polymere in besonderem Maße beeinträchtigen.

### Aufgabe der Erfindung

Aufgabe der Erfindung ist es, die oben genannten Nachteile zu überwinden und ein verbessertes Verfahren zur Herstellung von Methacrylsäure und / oder Alkylmethacrylaten basierend auf dem ACH-Sulfo-Verfahren bereitzustellen, bei welchem die Menge an störenden Nebenprodukten, insbesondere an Methacrylnitril (MAN) und Aceton, reduziert werden kann. Hierdurch soll die Produktqualität des Alkylmethacrylats sowie der daraus hergestellten Polymere und Formkörper verbessert werden. Insbesondere sollen die Verarbeitbarkeit sowie die mechanischen und optischen Eigenschaften der Alkylmethacrylat-Polymere verbessert werden.

Außerdem soll im Vergleich zu bekannten Verfahren eine vergleichbare oder erhöhte Ausbeute an Alkylmethacrylat erhalten werden.

### Lösung der Aufgabe

Die Aufgabe wurde insbesondere dadurch gelöst, dass im erfindungsgemäßen Verfahren die Bildung der genannten Nebenprodukte durch eine optimierte Reaktionsführung bei der Alkylmethacrylat-Synthese minimiert wird.

Es wurde überraschend gefunden, dass die oben genannten Aufgaben durch das erfindungsgemäße Verfahren gelöst werden. Insbesondere wurde gefunden, dass die Menge an störenden Nebenprodukten, insbesondere Methacrylnitril (MAN), Aceton, Methylpropionat und / oder Methylisobutyrat, reduziert werden kann, wenn bei der Umsetzung von Acetoncyanhydrin (Amidierung und Konvertierung) Schwefelsäure verwendet wird, die kein freies SO₃, insbesondere geringe Anteile freies Wasser enthält. Besonders vorteilhaft hat sich der Einsatz einer Schwefelsäure mit einer Konzentration im Bereich von 98,0 Gew.-% bis 100,0 Gew.-%, bevorzugt 99,0 Gew.-% bis 99,9 Gew.-%, erwiesen. Unter einer Schwefelsäure mit einer Konzentration von zum Beispiel 98,0 Gew.-% wird insbesondere ein Schwefelsäure-Reaktant oder ein Schwefelsäure-Zufuhrstrom verstanden, der zu 98,0 Gew.-% aus Schwefelsäure besteht, bezogen auf den gesamten Schwefelsäure-Reaktant bzw. Schwefelsäure-Zufuhrstrom. Insbesondere besteht der Schwefelsäure-Reaktant bzw. Schwefelsäure-Zufuhrstrom aus Schwefelsäure und Wasser. Ein weiterer Aspekt, der zur Lösung der beschriebenen Probleme maßgeblich beiträgt, ist die Kontrolle und Einstellung der Wassergehalte in den Einsatzstoffen Aceton sowie im Zwischenprodukt Acetoncyanhydrin. Allgemein gesagt, erlaubt das erfindungsgemäße Verfahren eine gezielte Herstellung und Kontrolle der Qualität und Zusammensetzung der Rohstoffe und Zwischenprodukte.

Weiterhin können die störenden Nebenprodukte, insbesondere Methacrylnitril (MAN) und Aceton, durch eine optimierte Aufarbeitung der Reaktionsmischung nach der Veresterung, umfassend eine geeignete Ausschleusung und eine optimierte Kreislaufführung von Verfahrensströmen, effektiv im erforderlichen Maß aus dem Verfahren ausgeschleust werden. Insbesondere wurde gefunden, dass sich eine effektive Ausschleusung von Methacrylnitril (MAN) und Aceton realisieren lässt, indem in mindestens einem Azeotrop-Destillationsschritt die Nebenprodukte zumindest teilweise in der Kopf-Fraktion als wasserhaltiges Heteroazeotrop erhalten und hierüber, gegebenenfalls nach weiteren Trennschritten, zumindest teilweise aus dem Verfahren ausgeschleust werden. Gegebenenfalls kann das Heteroazeotrop enthaltend die störenden Nebenprodukte in eine wässrige und eine organische Phase aufgetrennt werden, wobei die wässrige und / oder die organische Phase zumindest teilweise aus dem Verfahren ausgeschleust werden. Hierbei können die störenden Nebenprodukte zusammen mit solchen Stoffströmen aus dem Verfahren entfernt werden, in denen eine Anreicherung der störenden Nebenprodukte aufgrund ihrer physikochemischen Eigenschaften (insbesondere Wasserlöslichkeit und Flüchtigkeit) nicht zu erwarten ist. Weiterhin können durch Kombination mehrfacher Destillations- und Extraktionsschritte die störenden Nebenprodukte, insbesondere Aceton und Methacrylnitril, als Derivate aus dem Verfahren ausgeschleust werden (z.B. Aceton in sulfonierter Form) oder zu Zielprodukt umgewandelt werden (z.B. MAN über MASA in MMA).

Insgesamt gelingt es mit Hilfe des erfindungsgemäßen Verfahrens, das technische ACH-Sulfo-Verfahren robuster, weniger störungsanfällig und mit höheren Ausbeuten durchzuführen, wobei die Abtrennung von Alkylmethacrylaten in der erforderlichen Qualität effektiv möglich ist.

### Beschreibung der Erfindung

Die vorliegende Erfindung betrifft ein Verfahren zur Herstellung von Alkylmethacrylat, bevorzugt Methylmethacrylat, umfassend
a. Umsetzung von Aceton und Blausäure in Gegenwart eines basischen Katalysators in einer ersten Reaktionsstufe, wobei eine erste Reaktionsmischung enthaltend Acetoncyanhydrin (ACH) erhalten wird;
b. Aufarbeitung der ersten Reaktionsmischung enthaltend Acetoncyanhydrin (ACH);
c. Umsetzung von Acetoncyanhydrin und Schwefelsäure in einem oder mehreren Reaktoren I in einer zweiten Reaktionsstufe (Amidierung) bei einer Amidierungs-Temperatur im Bereich von 85 °C bis 130 °C, wobei eine zweite Reaktionsmischung, enthaltend Sulfoxyisobuttersäureamid und Methacrylamid, erhalten wird;
d. Konvertieren der zweiten Reaktionsmischung, umfassend Erhitzen auf eine Konvertierungs-Temperatur im Bereich von 130 °C bis 200 °C, in einem oder mehreren Reaktoren II in einer dritten Reaktionsstufe (Konvertierung), wobei eine dritte Reaktionsmischung, enthaltend überwiegend Methacrylamid (MASA) und Schwefelsäure, erhalten wird;
e. Umsetzung der dritten Reaktionsmischung mit Wasser und optional Alkohol, bevorzugt Wasser und optional Methanol, in einem oder mehreren Reaktoren III in einer vierten Reaktionsstufe (Hydrolyse oder Veresterung), wobei eine vierte Reaktionsmischung, enthaltend Methacrylsäure und / oder Alkylmethacrylat, bevorzugt Methylmethacrylat, erhalten wird;
f. gegebenenfalls Abtrennung von Alkylmethacrylat aus der vierten Reaktionsmischung erhalten aus der vierten Reaktionsstufe;
wobei die in der zweiten Reaktionsstufe eingesetzte Schwefelsäure eine Konzentration im Bereich von 98,0 Gew.-% bis 100,0 Gew.-% aufweist, wobei in der zweiten Reaktionsstufe eine Rein-ACH-Mischung eingesetzt wird, die einen Wassergehalt in einem Bereich von 0,1 mol-% bis 10 mol-%, insbesondere 0,4 mol-% bis 5 mol-% aufweist, bezogen auf in der Rein-ACH-Mischung enthaltenes ACH, und wobei in der zweiten Reaktionsstufe eine Gesamtmenge an Wasser in einem Bereich von 0,1 mol-% bis 20 mol-%, insbesondere 0,4 mol-% bis 10 mol-% eingesetzt wird, bezogen auf in der Rein-ACH-Mischung enthaltenes ACH. Insbesondere ist die Gesamtmenge an Wasser die Summe an Wasser, das mit der Rein-ACH-Mischung und gegebenenfalls mit der Schwefelsäure der zweiten Reaktionsstufe zugeführt wird.

Bevorzugt wird die vierte Reaktionsmischung, enthaltend Alkylmethacrylat, die in der vierten Reaktionsstufe erhalten wird, in weiteren Schritten umfassend mindestens einen Destillationsschritt und / oder mindestens einen Extraktionsschritt aufgearbeitet. Weiter bevorzugt wird die vierte Reaktionsmischung, enthaltend Alkylmethacrylat, die in der vierten Reaktionsstufe erhalten wird, in gasförmiger Form in einen Destillationsschritt geführt, wobei eine Kopf-Fraktion, enthaltend Alkylmethacrylat, Wasser und Alkohol, und eine Sumpf-Fraktion enthaltend schwerer siedende Komponenten, erhalten werden, und wobei die Sumpf-Fraktion vollständig oder teilweise in die vierte Reaktionsstufe zurückgeführt wird. Die Kopf-Fraktion, enthaltend Alkylmethacrylat, Wasser und Alkohol, wird bevorzugt in einem Phasentrennungsschritt oder nach einem Extraktionsschritt in eine organische Phase, enthaltend den überwiegenden Teil des Alkylmethacrylats, und in eine wässrige Phase, enthaltend Alkohol und weitere wasserlösliche Verbindungen, getrennt, wobei die wässrige Phase vollständig oder teilweise in die vierte Reaktionsstufe zurückgeführt wird und die organische Phase, enthaltend den überwiegenden Teil des Alkylmethacrylats, optional einer Extraktion unter Verwendung von Wasser als Extraktionsmittel unterzogen wird. Ferner wird bevorzugt die wässrige Phase dieser Extraktion in die vierte Reaktionsstufe zurückgeführt.

Im Sinne der vorliegenden Erfindung meint der Ausdruck "ppm", ohne weitere Angaben, Gew.-ppm (z.B. mg/kg).

Der Ausdruck Strom, Phase oder Fraktion enthalten ein Edukt, Produkt und / oder Nebenprodukt ist im Sinne der Erfindung so zu verstehen, dass die genannte(n) Verbindung(en) in dem jeweiligen Strom enthalten ist (sind), beispielsweise ist der überwiegende Anteil des Edukts, Produkts und / oder Nebenprodukts in dem entsprechenden Strom zu finden. Grundsätzlich können neben den genannten Verbindungen weitere Bestandteile enthalten sein. Oftmals dient die Nennung der Bestandteile der Verdeutlichung des jeweiligen Verfahrensschritts.

Der Ausdruck Brüden oder Brüdenstrom bezeichnet im Sinne der Erfindung einen gasförmigen Verfahrensstrom, beispielsweise einen gasförmigen Kopfstrom einer Destillationskolonne. Ein gasförmiger Brüdenstrom wird üblicherweise durch Kondensation und Kühlung verflüssigt. Im Falle eines heteroazeotropen Brüdens zerfällt diese Mischung üblicherweise in zwei Phasen, eine überwiegend organische Phase und eine wässrige, oft methanolische Phase.

### Erste Reaktionsstufe (Synthese von ACH)

Das erfindungsgemäße Verfahren umfasst als Schritt a. die Umsetzung von Aceton und Blausäure in Gegenwart eines basischen Katalysators in einer ersten Reaktionsstufe, wobei eine erste Reaktionsmischung enthaltend Acetoncyanhydrin (ACH) erhalten wird.

Bevorzugt wird in der ersten Reaktionsstufe ein Aceton-Reaktant eingesetzt wird, der 0,1 Gew.-% bis 1 Gew.-%, insbesondere 0,1 Gew.-% bis 0,5 Gew.-%, Wasser enthält, bezogen auf den gesamten Aceton-Reaktanten. Weiter bevorzugt enthält der Aceton-Reaktant 99 Gew.-% bis 99,9 Gew.-%, insbesondere 99,5 Gew.-% bis 99,9 Gew.-% Aceton, bezogen auf den gesamten Aceton-Reaktanten.

Weiterhin wird in der ersten Reaktionsstufe bevorzugt ein Blausäure-Reaktant eingesetzt, der 0,01 Gew.-% bis 0,1 Gew.-% Wasser enthält, bezogen auf den gesamten Blausäure-Reaktanten. Weiter bevorzugt enthält der Blausäure-Reaktant mindestens 99,9 Gew.-% Blausäure, bezogen auf den gesamten Blausäure-Reaktanten. Ein höherer Wasser-Gehalt im Blausäure-Reaktanten kann im Rahmen der Blausäure-Herstellung bei einer destillativen Blausäure-Aufreinigung, bei der auch Wasser abgetrennt wird, dazu führen, dass als Nebenprodukt auftretende Nitrile nicht gezielt ausgeschleust werden können und so ebenfalls in den Blausäure-Reaktanten gelangen. Ein geringerer Wasser-Gehalt ist ebenfalls nachteilig, da bei der destillativen Blausäure-Aufreinigung die genannten Nitrile in der Destillationskolonne verbleiben und dort polymerisieren können, was zu Verblockungen führt.

Das Acetoncyanhydrin (ACH) kann beispielsweise hergestellt werden wie in Ullmanns Enzyklopädie der technischen Chemie, 4. Auflage, Band 7, beschrieben. Der basische Katalysator ist bevorzugt ein Amin, wobei die Umsetzung von Aceton und Blausäure zu ACH eine exotherme Reaktion ist. Eine solche Verfahrensstufe ist beispielsweise in DE 10 2006 058 250 und DE 10 2006 059 511 näher beschrieben. Es sind alternative Verfahren beschrieben, die als Katalysator anstelle der organischen Amine Alkalihydroxid oder andere Alkaliderivate einsetzen. Alkaliverbindungen katalysieren zwar die ACH-Bildung effektiv, können aber bei den Folgeumsetzungen, insbesondere der Schwefelsäureregenerierung, zu Problemen wie z.B. Ablagerungen führen.

Um einer Zersetzung des in der ersten Reaktionsstufe gebildeten Acetoncyanhydrins entgegenzuwirken, wird üblicherweise die Reaktionswärme durch eine geeignete Vorrichtung abgeführt. Die erste Reaktionsstufe kann als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Ein Hauptmerkmal einer in hohen Ausbeuten zum gewünschten Produkt führenden Fahrweise liegt oft darin, dass bei ausreichender Reaktionszeit das Reaktionsprodukt gekühlt wird und das Reaktionsgleichgewicht in Richtung des Reaktionsproduktes verschoben wird. Darüber hinaus kann das Reaktionsprodukt der Umsetzung von Acton und Blausäure, das als erste Reaktionsmischung bezeichnet wird, zum Vorteil der Gesamtausbeute mit einem Stabilisator versetzt werden, um einer Zersetzung bei der späteren Aufarbeitung vorzubeugen.

Die Vermischung der Reaktionspartner Aceton und Blausäure kann grundsätzlich auf im Wesentlichen beliebige Weise erfolgen. Die Art der Vermischung hängt insbesondere davon ab, ob eine diskrete Fahrweise, beispielsweise im Batchreaktor, oder eine kontinuierliche Fahrweise, beispielsweise im Schlaufenreaktor, gewählt wird.

Grundsätzlich kann es vorteilhaft sein, wenn das Aceton in die Reaktion über mindestens einen Vorlagebehälter eingespeist wird, der bevorzugt über einen Waschturm verfügt. Entlüftungsleitungen, die Aceton und Blausäure enthaltende Abluft führen, können so beispielsweise durch diesen Vorlagebehälter geführt werden. Im Waschturm, der an den Vorlagebehälter angeschlossen ist, kann die aus dem Vorlagebehälter entweichende Abluft mit Aceton gewaschen werden, wodurch Blausäure aus der Abluft entfernt und in den Prozess rückgeführt wird. Hierzu wird beispielsweise ein Teil der vom Vorlagebehälter in die Reaktion eingeführten Acetonmenge im Teilstrom über einen Kühler, vorzugsweise über einen Sole-Kühler, in den Kopf des Waschturms geführt und so das gewünschte Ergebnis erzielt.

Die Temperatur des Acetons im Vorlagebehälter kann grundsätzlich innerhalb eines im Wesentlichen beliebigen Bereiches liegen, insofern das Aceton sich bei der entsprechenden Temperatur im flüssigen Zustand befindet. Vorteilhafterweise beträgt die Temperatur im Vorlagebehälter jedoch etwa 0 bis etwa 20 °C.

Im Waschturm wird das zum Waschen eingesetzte Aceton über einen entsprechenden Kühler, beispielsweise über einen Plattenkühler mit Sole auf eine Temperatur von etwa 0 bis etwa 10 °C gekühlt. Die Temperatur des Acetons beim Eintritt in den Waschturm beträgt daher vorzugsweise beispielsweise etwa 2 bis etwa 6 °C.

Die in der ersten Reaktionsstufe benötigte Blausäure kann entweder in flüssiger oder in gasförmiger Form in den Reaktor eingeführt werden. Es kann sich dabei beispielsweise um Rohgas aus dem BMA- oder aus dem Andrussow-Prozess handeln.

Die Blausäure kann beispielsweise verflüssigt werden, beispielsweise durch den Einsatz einer entsprechenden Kühlsole. Anstelle von verflüssigter Blausäure kann Kokereigas eingesetzt werden. So werden beispielsweise Cyanwasserstoff enthaltende Kokereigase nach einer Wäsche mit Pottasche kontinuierlich im Gegenstrom mit Aceton gewaschen, und die Reaktion zu Acetoncyanhydrin kann in Gegenwart eines basischen Katalysators in zwei hintereinander geschalteten Gaswaschkolonnen durchgeführt werden.

Im Rahmen einer weiteren Ausführungsform kann ein Cyanwasserstoff und inerte Gase enthaltendes Gasgemisch, insbesondere ein Rohgas aus dem BMA- oder aus dem Andrussow-Prozess, mit Aceton in Gegenwart eines basischen Katalysators und Acetoncyanhydrin in einem Gas-Flüssig-Reaktor umgesetzt werden.

Bevorzugt wird in der ersten Reaktionsstufe ein BMA-Rohgas oder ein Andrussow-Rohgas eingesetzt. Das aus den oben genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im Wesentlichen Blausäure und Wasserstoff gebildet werden, enthält typischerweise 22,9 Vol.-% HCN, 71,8 Vol.-% H₂, 2,5 Vol.-% NH₃, 1,1 Vol.-% N₂, 1,7 Vol.-% CH₄. Im bekannten Andrussow-Prozess werden aus Methan, Ammoniak und Luftsauerstoff Blausäure und Wasser gebildet. Das Rohgas des Andrussow-Prozesses enthält beim Einsatz von Sauerstoff als Sauerstoffquelle typischerweise 8 Vol.-% HCN, 22 Vol.-% H₂O, 46,5 Vol.-% N₂, 15 Vol.-% H₂, 5 Vol.-% CO, 2,5 Vol.-% NH₃ und je 0,5 Vol.-% CH₄ und CO₂.

Beim Einsatz eines nicht säuregewaschenen Rohgases aus dem BMA- oder Andrussow-Prozess wirkt der im Rohgas enthaltene Ammoniak häufig als Katalysator für die Reaktion. Da der im Rohgas enthaltene Ammoniak häufig die als Katalysator erforderliche Menge übersteigt und deshalb zu Verlusten an zur Stabilisierung eingesetzter Schwefelsäure führen kann, kann ein derartiges Rohgas oft einer Säurewäsche unterzogen werden, um Ammoniak daraus zu eliminieren. Beim Einsatz eines solchen mit Säure gewaschenen Rohgases wird bevorzugt ein geeigneter basischer Katalysator dem Reaktor der ersten Reaktionsstufe in katalytischer Menge zugesetzt. Im Prinzip können dabei bekannte anorganische oder organische basische Verbindungen als Katalysator fungieren.

Blausäure in gasförmiger oder in flüssiger Form bzw. ein Blausäure enthaltendes Gasgemisch und Aceton werden im Rahmen der kontinuierlichen Fahrweise fortlaufend insbesondere einem Schlaufenreaktor zugeführt. Der Schlaufenreaktor umfasst dabei bevorzugt mindestens eine Möglichkeit zum Zuführen von Aceton oder zwei oder mehr solcher Möglichkeiten, mindestens eine Möglichkeit zur Zuführung von flüssiger oder gasförmiger Blausäure, oder zwei oder mehr solcher Möglichkeiten sowie mindestens eine Möglichkeit zum Zuführen des Katalysators.

Als Katalysator eignen sich grundsätzlich beliebige alkalische Verbindungen wie Ammoniak, Natronlauge oder Kalilauge, die die Umsetzung von Aceton und Blausäure zu Acetoncyanhydrin katalysieren können. Es hat sich jedoch als vorteilhaft herausgestellt, wenn als Katalysator ein organischer Katalysator, insbesondere ein Amin eingesetzt wird. Geeignet sind beispielsweise sekundäre oder tertiäre Amine, wie Diethylamin, Dipropylamin, Triethylamin, Tri-n-propylamin und dergleichen.

Ein in der ersten Reaktionsstufe einsetzbarer Schlaufenreaktor weist bevorzugt mindestens eine Pumpe und mindestens eine Mischvorrichtung auf. Als Pumpe eignen sich grundsätzlich alle Pumpen, die geeignet sind, den Umlauf des ersten Reaktionsgemisches im Schlaufenreaktor zu gewährleisten. Als Mischvorrichtungen sind sowohl Mischvorrichtungen mit beweglichen Elementen als auch sogenannte Statikmischer geeignet, bei denen unbewegliche Strömungswiderstände vorgesehen sind. Im Falle des Einsatzes von Statikmischern sind beispielsweise solche geeignet, die einen Betriebsüberdruck von mindestens 10, beispielsweise mindestens 15 oder mindestens 20 bar unter Betriebsbedingungen ohne wesentliche Einschränkungen der Funktionsfähigkeit erlauben. Entsprechende Mischer können aus Kunststoff oder Metall bestehen. Als Kunststoff eignen sich beispielsweise PVC, PP; HDPE, PVDF, PFA oder PTFE. Metallmischer können beispielsweise aus Nickellegierungen, Zirkonium, Titan und dergleichen bestehen. Ebenfalls geeignet sind beispielsweise Rechteckmischer.

Die Zugabe des Katalysators erfolgt in der ersten Reaktionsstufe vorzugsweise im Schlaufenreaktor nach der Pumpe und vor einem im Schlaufenreaktor vorhandenen Mischelement. Katalysatoren werden beispielsweise in einer solchen Menge eingesetzt, dass die Gesamtreaktion in der ersten Reaktionsstufe bei einem pH-Wert von maximal 8, insbesondere maximal 7,5 oder 7 gefahren wird. Es ist bevorzugt, daß sich der pH-Wert bei der Reaktion in der ersten Reaktionsstufe innerhalb eines Bereichs von 6,5 bis etwa 7,5, beispielsweise 6,8 bis 7,2 liegt.

Es ist in der ersten Reaktionsstufe alternativ zur Zugabe des Katalysators in den Schlaufenreaktor nach der Pumpe und vor einer Mischvorrichtung auch möglich, den Katalysator zusammen mit dem Aceton in den Schlaufenreaktor einzuspeisen. In einem solchen Fall kann es vorteilhaft sein, wenn für eine entsprechende Vermischung von Aceton und Katalysator vor Einspeisung in den Schlaufenreaktor gesorgt wird. Eine entsprechende Vermischung kann beispielsweise durch den Einsatz eines Mischers mit beweglichen Teilen oder durch Einsatz eines Statikmischers erfolgen. Dies erfolgt im Wesentlichen unter Beachtung von Temperaturen und Verweilzeiten, um unerwünschte Verbrauchsreaktionen des Katalysators (z.B. die Cannizzaro-Reaktion) oder Kondensationsreaktionen des Acetons zu unterdrücken und zu verhindern. Um dies zu gewährleisten, wird bei möglichst kurzen Verweilzeiten und kalt gearbeitet.

Wenn in der ersten Reaktionsstufe eine kontinuierliche Fahrweise in einem Schlaufenreaktor als Betriebsart gewählt wird, so kann es zweckmäßig sein, den Zustand des ersten Reaktionsgemischs durch punktuelle oder fortlaufende Analysen zu untersuchen. Dies bietet den Vorteil, dass gegebenenfalls auch auf Zustandsänderungen im ersten Reaktionsgemisch schnell reagiert werden kann. Darüber hinaus lassen sich so beispielsweise die Reaktionspartner möglichst genau dosieren, um Ausbeuteverluste zu minimieren.

Eine entsprechende Analytik kann beispielsweise durch Probennahme in der Reaktorschlaufe erfolgen. Geeignete Analyseverfahren sind beispielsweise pH-Messung, Messung der Wärmetönung oder Messung der Zusammensetzung des ersten Reaktionsgemisches durch geeignete spektroskopische Verfahren.

Insbesondere im Rahmen der Umsatzkontrolle, Qualitätsaspekten und Sicherheit hat es sich bewährt, den Umsatz im ersten Reaktionsgemisch über die aus dem ersten Reaktionsgemisch abgeführte Wärme zu bestimmen und mit der theoretisch freiwerdenden Wärme zu vergleichen.

Die eigentliche Reaktion der ersten Reaktionsstufe kann bei geeigneter Wahl des Schlaufenreaktors grundsätzlich in den innerhalb des Schlaufenreaktors angeordneten Rohrsystemen erfolgen. Da die Reaktion jedoch exotherm ist, sollte, um Ausbeuteverlust zu vermeiden, auf eine ausreichende Kühlung bzw. auf eine ausreichende Abfuhr der Reaktionswärme geachtet werden. Es hat sich häufig als vorteilhaft erwiesen, wenn die erste Reaktionsstufe in einem Wärmetauscher, vorzugsweise in einem Rohrbündelwärmetauscher, ausgeführt wird. Je nach zu produzierender Menge kann die Kapazität eines entsprechenden Wärmetauschers unterschiedlich gewählt werden.

Bei den bevorzugt eingesetzten Rohrbündelwärmetauschern handelt es sich um Wärmetauscher, die in einem flüssigkeitsdurchströmten Mantel ein flüssigkeitsdurchströmtes Rohrbündel aufweisen. Je nach Rohrdurchmesser, Packungsdichte, etc. kann der Wärmeübergang zwischen den beiden Flüssigkeiten entsprechend eingestellt werden. Es ist in der ersten Reaktionsstufe grundsätzlich möglich, die Reaktion dahingehend zu führen, dass das erste Reaktionsgemisch durch den Wärmetauscher im Rohrbündel selbst gefahren wird und die Reaktion innerhalb des Rohrbündels stattfindet, wobei die Wärme aus dem Rohrbündel in die Mantelflüssigkeit abgeführt wird.

Das erste Reaktionsgemisch kann alternativ durch den Mantel des Wärmetauschers geführt werden, während die zur Kühlung eingesetzte Flüssigkeit innerhalb des Rohrbündels zirkuliert. Dabei hat es sich in vielen Fällen als vorteilhaft erwiesen, wenn die erste Reaktionsmischung im Mantel zur Erzielung einer höheren Verweildauer und einer besseren Durchmischung über Strömungswiderstände, vorzugsweise Umlenkbleche, verteilt wird.

Das Verhältnis von Mantelvolumen zum Volumen des Rohrbündels kann dabei zum Beispiel, je nach Auslegung des Reaktors, 10 zu 1 bis 1 zu 10 betragen, vorzugsweise ist das Volumen des Mantels größer als das Volumen des Rohrbündels (bezogen auf den Inhalt der Rohre).

Die Wärmeabfuhr aus dem Reaktor in der ersten Reaktionsstufe wird bevorzugt mit einem Kühlmittel, beispielsweise mit Wasser, so eingestellt, dass die Reaktionstemperatur (Synthesetemperatur) in der ersten Reaktionsstufe in einem Bereich von 25 bis 45 °C, weiter bevorzugt von 30 bis 38 °C, insbesondere von 33 bis 35 °C liegt.

Aus dem Schlaufenreaktor der ersten Reaktionsstufe wird bevorzugt kontinuierlich ein Produkt, insbesondere die erste Reaktionsmischung, abgeführt. Die erste Reaktionsmischung weist bevorzugt eine Temperatur im Rahmen der oben genannten Synthesetemperatur, beispielsweise eine Temperatur von etwa 35°C auf. Das Produkt, insbesondere die erste Reaktionsmischung, wird bevorzugt über einen oder mehrere Wärmetauscher, insbesondere über einen oder mehrere Plattenwärmetauscher gekühlt. Dabei kommt beispielsweise eine Solekühlung zum Einsatz. Die Temperatur des Produkts, insbesondere der ersten Reaktionsmischung, nach Kühlung beträgt bevorzugt 0 bis 10 °C, insbesondere 1 bis 5 °C. Das Produkt, insbesondere die erste Reaktionsmischung, wird vorzugsweise in einen Lagerbehälter überführt, der eine Pufferfunktion aufweist. Zusätzlich kann das Produkt, insbesondere die erste Reaktionsmischung, im Lagerbehälter beispielsweise durch ständiges Abführen eines Teilstroms aus dem Lagerbehälter zu einem geeigneten Wärmetauscher, beispielsweise zu einem Plattenwärmetauscher, weiter gekühlt werden, bzw. auf einer geeigneten Lagertemperatur gehalten werden. Es ist durchaus möglich, dass in dem Lagerbehälter eine Nachreaktion stattfinden kann. Üblicherweise findet je nach Temperatur und Verweilzeit im Tank eine Nachreaktion gemäß der thermodynamischen Lage des ACH-Gleichgewichts mit den Edukten statt, da der Katalysator noch in aktiver Form vorliegt. Soll diese Reaktion unterdrückt werden, muß der Katalysator neutralisiert werden.

Die Rückführung des Produkts, insbesondere der ersten Reaktionsmischung, in den Lagerbehälter, kann grundsätzlich auf beliebige Weise erfolgen. Es hat sich jedoch in einigen Fällen als vorteilhaft herausgestellt, dass das Produkt, insbesondere die erste Reaktionsmischung, derart über ein System aus einer oder mehreren Düsen in den Lagerbehälter rückgeführt wird, dass innerhalb des Lagerbehälters eine entsprechende Durchmischung des gelagerten Produktes, insbesondere der ersten Reaktionsmischung, stattfindet.

Aus dem Lagerbehälter wird weiter bevorzugt kontinuierlich Produkt, insbesondere erste Reaktionsmischung, in einen Stabilisierungsbehälter abgeführt. Dort wird das Produkt, insbesondere die erste Reaktionsmischung, mit einer geeigneten Säure, beispielsweise mit H₂SO₄, versetzt. Dabei wird der Katalysator deaktiviert und die erste Reaktionsmischung auf einen pH-Wert von etwa 1 bis etwa 3, insbesondere etwa 2 eingestellt. Als Säure eignet sich insbesondere Schwefelsäure, beispielsweise Schwefelsäure mit einem Gehalt von etwa 10 bis etwa 105 %, insbesondere von etwa 80 bis etwa 98 % H₂SO₄. Dies ist hinsichtlich des erfindungsgemäßen Verfahrens in mehrfacher Hinsicht wichtig, zumal die Stabilisatorsäure auch ein Reaktant der nachfolgenden Amidierung ist sowie Katalysator der Veresterung und Verseifung. Zum anderen nimmt die Schwefelsäure-Konzentration Einfluß auf das Wassermanagement des Gesamtprozesses und auch auf den Wassergehalt im ACH.

Das stabilisierte Produkt, insbesondere die stabilisierte erste Reaktionsmischung, wird der ersten Reaktionsstufe, insbesondere dem Stabilisierungsbehälter, entnommen und in die Aufarbeitungsstufe überführt. Dabei kann ein Teil des entnommenen, stabilisierten Produkts, insbesondere der ersten Reaktionsmischung, beispielsweise derart in den Stabilisierungsbehälter zurückgeführt werden, dass eine ausreichende Durchmischung des Behälters über ein System aus einer oder mehreren Düsen gewährleistet ist.

### Aufarbeitung des synthetisierten ACH

Als Schritt b. umfasst das erfindungsgemäße Verfahren die Aufarbeitung der ersten Reaktionsmischung, enthaltend Acetoncyanhydrin (ACH). Bevorzugt umfasst die Aufarbeitung der ersten Reaktionsmischung in Schritt b. einen Destillationsschritt, wobei Acetoncyanhydrin zumindest teilweise von Verunreinigungen und / oder nicht umgesetzten Ausgangsstoffen und / oder Nebenprodukten, die niedrigsiedender sind als Acetoncyanhydrin, abgetrennt wird.

Im Rahmen der Aufarbeitung wird die erste Reaktionsmischung, die in der vorgelagerten ersten Reaktionsstufe erhalten wurde, bevorzugt einer destillativen Aufarbeitung zugeführt. Dabei wird die, insbesondere stabilisierte, erste Reaktionsmischung bevorzugt über eine entsprechende Kolonne von niedrigsiedenden Bestandteilen befreit. Ein geeignetes Destillationsverfahren kann beispielsweise über nur eine Kolonne geführt werden. Es ist jedoch ebenfalls möglich, im Rahmen der Aufarbeitung der ersten Reaktionsmischung eine Kombination von zwei oder mehr Destillationskolonnen auch kombiniert mit einem Fallfilmverdampfer einzusetzen. Weiterhin können zwei oder mehrere Fallfilmverdampfer oder auch zwei oder mehrere Destillationskolonnen miteinander kombiniert werden.

Die erste Reaktionsmischung kommt bevorzugt mit einer Temperatur in einem Bereich von 0 bis 15 °C, beispielsweise von 5 bis 10 °C aus der Lagerung zur Destillation. Die erste Reaktionsmischung kann direkt in die Kolonne eingeführt werden. Es hat sich jedoch in einigen Fällen bewährt, wenn zunächst die kühle erste Reaktionsmischung über einen Wärmetauscher einen Teil der Wärme der bereits destillativ gereinigten ersten Reaktionsmischung, die auch als Rein-ACH-Mischung bezeichnet wird, übernimmt. Dies wird durch Wärmetausch zwischen Sumpf der Kolonne mit dem Feedgemisch über entsprechende Apparate erreicht. Daher wird im Rahmen einer weiteren Ausführungsform die erste Reaktionsmischung über einen Wärmetauscher auf eine Temperatur in einem Bereich von 60 bis 80 °C erhitzt.

Die destillative Reinigung erfolgt insbesondere über eine Destillationskolonne, vorzugsweise mit mehr als 10 Böden oder über eine Kaskade von zwei oder mehr entsprechend geeigneten Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140 °C nicht übersteigt; gute Ausbeuten und eine gute Reinigung haben sich erzielen lassen, wenn die Sumpftemperatur nicht größer als etwa 130 °C oder nicht höher als etwa 110 °C ist. Die Temperaturangaben beziehen sich dabei auf die Wandtemperatur des Kolonnensumpfs.

Die erste Reaktionsmischung wird bevorzugt im oberen Drittel der Kolonne dem Kolonnenkörper zugeführt. Die Destillation wird vorzugsweise bei verringertem Druck, beispielsweise bei einem Druck von 50 bis 900 mbar, insbesondere von 50 bis 250 mbar und mit guten Ergebnissen zwischen 50 bis 150 mbar durchgeführt.

Am Kopf der Kolonne werden bevorzugt gasförmige Edukte (HCN und Aceton und Spuren an Wasser) und Verunreinigungen, insbesondere Aceton und Blausäure, entnommen; die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von 0 bis 10 °C eingesetzt. Dabei werden die gasförmigen Inhaltsstoffe der Brüden zumindest partial kondensiert. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von 0 bis 15 °C, insbesondere bei 5 bis 10 °C gesammelt.

Die im Rahmen des ersten Kondensationsschritts nicht kondensierenden gasförmigen Verbindungen werden bevorzugt über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Ein auf der Druckseite der Pumpe entweichende Gasstrom wird bevorzugt über einen weiteren Wärmetauscher geführt, der vorzugsweise mit Sole bei einer Temperatur von 0 bis 15 °C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell und/oder parallel angeordneten Wärmetauschern erfolgen. Nach diesem Kondensationsschritt verbleibende gasförmige Stoffe werden abgeführt und einer beliebigen weiteren Verwertung, beispielsweise einer thermischen Verwertung, zugeführt.

Die gesammelten Kondensate können ebenfalls weiterverwertet werden. Es hat sich jedoch als unter ökonomischen Gesichtspunkten äußerst vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor der ersten Reaktionsstufe ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensats jedoch aus Aceton und Blausäure bestehen, beispielsweise in einem molaren Verhältnis von 2:1 bis 1:2, häufig in einem Verhältnis von etwa 1:1.

Wenn im Kopf der Kolonne Spuren an Amin mit kondensiert werden, kommt es in der flüssigen Phase im Bereich des Kondensators durch die flüssige Anwesenheit der Edukte HCN und Aceton zur Bildung von ACH. Dieses Kopfkondensat kann entweder in die Reaktion zurückgeführt werden oder anteilig als Rücklauf auf die Kolonne zurückgeführt werden.

Die aus dem Sumpf der Destillationskolonne gewonnene Rein-ACH-Mischung wird bevorzugt zunächst über einen ersten Wärmetauscher durch die zugeführte, kalte erste Reaktionsmischung auf eine Temperatur von 40 bis 80 °C abgekühlt. Anschließend wird die Rein-ACH-Mischung weiter bevorzugt über mindestens einen weiteren Wärmetauscher auf eine Temperatur von 30 bis 35 °C gekühlt und ggf. zwischengelagert.

### Zweite Reaktionsstufe (Amidierung)

Das erfindungsgemäße Verfahren umfasst als Schritt c. die Umsetzung von Acetoncyanhydrin und Schwefelsäure in einem oder mehreren Reaktoren I in einer zweiten Reaktionsstufe (Amidierung) bei einer Amidierungs-Temperatur im Bereich von 85 °C bis 130 °C, wobei eine zweite Reaktionsmischung, enthaltend Sulfoxyisobuttersäureamid und Methacrylamid, erhalten wird.

Erfindungsgemäß weist die in der zweiten Reaktionsstufe eingesetzte Schwefelsäure eine Konzentration im Bereich von 98,0 Gew.-% bis 100,0 Gew.-%, bevorzugt von 99,0 Gew.-% bis 99,9 Gew.-%, bevorzugt von 99,3 bis 99,9 Gew.-%, insbesondere bevorzugt von 99,3 bis 99,8 Gew.-% auf. Der Einsatz einer Schwefelsäure ohne Anteil an freiem SO₃, insbesondere einer Schwefelsäure mit einem Wasseranteil von 0,1 bis 0,7 Gew.-% hat sich als besonders vorteilhaft erwiesen. Insbesondere konnte hierdurch die Amidierungsausbeute erhöht und der Anteil an Nebenprodukten, insbesondere MAN und Aceton, vermindert werden.

Typischerweise entstehen bei der Umsetzung (Amidierung) von Acetoncyanhydrin (ACH) und Schwefelsäure als Hauptprodukte alpha-Hydroxyisobuttersäureamid (HIBA) bzw. dessen Hydrogensulfat (HIBA·H₂SO₄), Schwefelsäureester von alpha-Hydroxyisobuttersäureamid (Sulfoxyisobuttersäureamid (SIBA)) bzw. dessen Hydrogensulfat (SIBA·H₂SO₄) und Methacrylamid-Hydrogensulfat (MASA·H₂SO₄) als Lösung in überschüssiger Schwefelsäure. Durch die Gegenwart geringer und gemäß Erfindung definierter Mengen Wasser werden ebenfalls gewisse Konzentrationen an MAS und HIBS detektiert, in geringerem Umfang in der Amidierung selbst, in höheren Konzentrationen im nachfolgenden Schritt der Konvertierung.

Bevorzugt wird die Rein-ACH-Mischung, die der zweiten Reaktionsstufe zugeführt wird, in der Aufarbeitung in Schritt b. erhalten. Insbesondere wird die Rein-ACH-Mischung ausgehend von der Aufarbeitung in Schritt b. der zweiten Reaktionsstufe unverändert zugeführt. Weiter bevorzugt wird die Gesamtmenge an ACH, die der zweiten Reaktionsstufe zugeführt wird, mit der Rein-ACH-Mischung zugeführt. Die Rein-ACH-Mischung weist bevorzugt einen Acetongehalt von kleiner oder gleich 9.000 ppm, weiter bevorzugt von kleiner oder gleich 4.000 ppm, mehr bevorzugt von kleiner oder gleich 1.000 ppm, auf, bezogen auf die Gesamtmenge an ACH, die der zweiten Reaktionsstufe zugeführt wird. Bevorzugt weist die Rein-ACH-Mischung einen ACH-Anteil von größer oder gleich 98 Gew.-%, besonders bevorzugt größer oder gleich 98,5 Gew.-%, insbesondere bevorzugt größer oder gleich 99 Gew.-%, auf, bezogen auf die gesamte Rein-ACH-Mischung. Typischerweise enthält die in der zweiten Reaktionsstufe eingesetzte Rein-ACH-Mischung, insbesondere Stoffstrom (8a) bzw. (8b) und (8c), 98,0 bis 99,8 Gew.-%, bevorzugt 98,3 bis 99,5 Gew.-%, Acetoncyanhydrin; gegebenenfalls 0,1 bis 1,5 Gew.-%, bevorzugt 0,2 bis 1 Gew.-%, Aceton und Wasser, bezogen auf die gesamte Rein-ACH-Mischung, wobei die Gesamtsumme 100 % ergibt. Ferner kann die Rein-ACH-Mischung aus ACH und Wasser bestehen. Weiterhin ist der basische Katalysator, optional in neutralisierter Form, anwesend. Dies ist insbesondere der Fall, wenn die ACH-Reaktionsmischung als Sumpfprodukt der Aufreinigung anfällt.

Bevorzugt wird die zweite Reaktionsstufe mit einem Überschuss an Schwefelsäure betrieben. Die Schwefelsäure dient bevorzugt als Lösemittel, Reaktant und Katalysator. Der Schwefelsäure-Überschuss kann insbesondere dazu dienen, die Viskosität der zweiten Reaktionsmischung niedrig zu halten, wodurch eine schnellere Abfuhr von Reaktionswärme und eine niedrigere Temperatur der zweiten Reaktionsmischung gewährleistet werden können. Dies kann insbesondere deutliche Ausbeutevorteile mit sich bringen.

Bevorzugt werden Schwefelsäure und Acetoncyanhydrin (ACH) in der zweiten Reaktionsstufe in einem molaren Verhältnis von Schwefelsäure zu ACH im Bereich von 1,2 bis 2,0; weiter bevorzugt 1,2 bis 1,5; eingesetzt. Bevorzugt umfasst die zweite Reaktionsstufe mindestens zwei getrennte Reaktoren I, wobei Schwefelsäure und Acetoncyanhydrin (ACH) in einem ersten Reaktor I in einem molaren Verhältnis von Schwefelsäure zu ACH im Bereich von 1,6 bis 3; bevorzugt 1,7 bis 2,6; besonders bevorzugt von 1,8 bis 2,3; eingesetzt werden, bezogen auf im ersten Reaktor I eingesetztes ACH, und wobei Schwefelsäure und Acetoncyanhydrin (ACH) in einem letzten Reaktor I (beispielsweise in einem zweiten Reaktor I) in einem molaren Verhältnis von Schwefelsäure zu ACH im Bereich von 1,2 bis 2,0; bevorzugt von 1,2 bis 1,5; eingesetzt werden, bezogen auf eine Gesamtmenge an ACH, die der zweiten Reaktionsstufe zugeführt wird.

Die Umsetzung von Acetoncyanhydrin mit Schwefelsäure in der zweiten Reaktionsstufe ist exotherm. Daher ist es vorteilhaft, die anfallende Reaktionswärme weitgehend oder zumindest teilweise abzuführen, beispielsweise mit Hilfe geeigneter Wärmetauscher, um eine verbesserte Ausbeute zu erhalten. Da mit sinkender Temperatur die Viskosität der zweiten Reaktionsmischung stark ansteigt und so Zirkulation, Durchfluss und Wärmeaustausch in den Reaktoren I erschwert sind, ist eine zu starke Abkühlung allerdings zu vermeiden. Darüber hinaus kann es bei niedrigen Temperaturen in der zweiten Reaktionsmischung zu einer partiellen oder vollständigen Kristallisation von Inhaltsstoffen an den Wärmetauschern kommen, was zu Abrasion, beispielsweise in den Pumpengehäusen, Rohrleitungen und den Wärmetauscher-Rohren der Reaktoren I führen kann.

Zur Kühlung der Reaktorkreisläufe können grundsätzlich bekannte und geeignete Kühlmedien eingesetzt werden. Vorteilhaft ist der Einsatz von Kühlwasser. Typischerweise weist das Kühlmedium, insbesondere das Kühlwasser, eine Temperatur unterhalb der gewählten Verfahrensbedingungen auf. Vorteilhaft weist das Kühlmedium, insbesondere das Kühlwasser, eine Temperatur im Bereich von 20 bis 90 °C, bevorzugt von 50 bis 90 °C und besonders bevorzugt von 60 bis 70 °C auf.

Zur Vermeidung der Unterschreitung des Kristallisationspunkts von Methacrylamid und anderer in der Reaktionsmatrize vorhandener Salze wird der Wärmetauscher (Reaktorkühler) typischerweise mit einem Warmwasser-Sekundärkreislauf betrieben. Hierbei sind Temperaturdifferenzen im produktseitigen Ein- / Austritt des Apparats von etwa 1 bis 20 °C, insbesondere 2 bis 10 °C bevorzugt.

Die Umsetzung von Acetoncyanhydrin und Schwefelsäure in einem oder mehreren Reaktoren I in der zweiten Reaktionsstufe (Amidierung) erfolgt bei einer Amidierungs-Temperatur im Bereich von 85 °C bis 130 °C, bevorzugt von 85 °C bis 120 °C, besonders bevorzugt von 90 °C und 110 °C. Oftmals wird die Amidierung in der zweiten Reaktionsstufe im Reaktor I oder in mehreren Reaktoren I bei Normaldruck oder moderatem Unterdruck durchgeführt.

Typischerweise kann die zweite Reaktionsstufe (Amidierung) batchweise und / oder kontinuierlich durchgeführt werden. Bevorzugt wird die zweite Reaktionsstufe kontinuierlich, beispielsweise in einem oder in mehreren Schlaufenreaktoren, durchgeführt. Geeignete Reaktoren und Verfahren sind beispielsweise in WO 2013/143812 beschrieben. Vorteilhaft kann die zweite Reaktionsstufe in einer Kaskade von zwei oder mehreren Schlaufenreaktoren durchgeführt werden. Insbesondere bevorzugt erfolgt die Umsetzung der zweiten Reaktionsstufe in einem oder in mehreren (bevorzugt zwei) Schlaufenreaktoren.

Der erste Schlaufenreaktor wird typischerweise bei einem Kreislaufverhältnis (Verhältnis aus Umwälzvolumenstrom zu Zulaufvolumenstrom) im Bereich von 5 bis 110, bevorzugt bei 10 bis 90, besonders bevorzugt bei 10 bis 70 betrieben. In einem nachfolgenden Schlaufenreaktor liegt das Kreislaufverhältnis vorzugsweise in einem Bereich von 5 bis 100, bevorzugt von 10 bis 90, besonders bevorzugt von 10 bis 70.

Typischerweise liegt die statistische Verweilzeit in den Reaktoren I, insbesondere in den Schlaufenreaktoren I, im Bereich von 5 bis 35 Minuten, bevorzugt von 8 bis 20 Minuten.

Ein geeigneter Schlaufenreaktor weist bevorzugt die folgenden Elemente auf: eine oder mehrere Zugabestellen für ACH, eine oder mehrere Zugabestellen für Schwefelsäure, einen oder mehrere Gasabscheider, einen oder mehrere Wärmetauscher, einen oder mehrere Mischer, und eine Pumpe. Die Mischer sind häufig als statische Mischer ausgeführt.

Die Zugabe des ACH kann grundsätzlich an beliebiger Stelle in den einen oder in die mehreren Reaktoren I (z.B. Schlaufenreaktoren) erfolgen. Es hat sich jedoch als vorteilhaft erwiesen, wenn die Zugabe des ACH an einer gut durchmischten Stelle erfolgt. Bevorzugt erfolgt die Zugabe des ACH in ein Mischelement, beispielsweise in einen Mischer mit beweglichen Teilen oder in einen statischen Mischer.

Die Zugabe der Schwefelsäure kann grundsätzlich an beliebiger Stelle in den einen oder in die mehreren Rektoren I (z.B. Schlaufenreaktoren) erfolgen. Bevorzugt erfolgt die Zugabe der Schwefelsäure vor der Zugabe des ACH. Besonders bevorzugt erfolgt die Zugabe der Schwefelsäure auf der Saugseite der jeweiligen Reaktorpumpe. Oftmals kann hierdurch die Pumpfähigkeit der gashaltigen Reaktionsmischung verbessert werden.

Die Reaktoren I (z.B. die Schlaufenreaktoren I) umfassen bevorzugt jeweils mindestens einen Gasabscheider. Typischerweise kann über den Gasabscheider einerseits kontinuierlich Produktstrom (zweite Reaktionsmischung) entnommen werden, andererseits lassen sich gasförmige Nebenprodukte abtrennen und ausschleusen. Typischerweise bildet sich als gasförmiges Nebenprodukt hauptsächlich Kohlenstoffmonoxid. Bevorzugt wird ein Teil des Abgases, welches in der Amidierung erhalten wird, zusammen mit der dritten Reaktionsmischung, welche in der dritten Reaktionsstufe (Konvertierung) erhalten wird, in einen Gasabscheider geführt.

In einer bevorzugten Ausführungsform umfasst die zweite Reaktionsstufe die Umsetzung von Acetoncyanhydrin (ACH) und Schwefelsäure in mindestens zwei getrennten Reaktionszonen, bevorzugt in mindestens zwei Schlaufenreaktoren.

Bevorzugt erfolgt die Umsetzung von Acetoncyanhydrin (ACH) und Schwefelsäure in der Art, dass das Reaktionsvolumen auf mindestens zwei Reaktionszonen verteilt ist, und die Gesamtmenge an ACH in die verschiedenen Reaktionszonen separat dosiert wird. Bevorzugt ist die Menge an ACH, die dem ersten Reaktor oder der ersten Reaktionszone zugeführt wird, größer oder gleich der Mengen an ACH, die den nachfolgenden Reaktoren oder den nachfolgenden Reaktionszonen zugeführt werden.

Vorzugsweise werden 50-90 Gew.-%, bevorzugt 60 bis 75 Gew.-%, des gesamten Volumenstroms an zugeführtem ACH, in den ersten Reaktor eingeführt. Die verbleibende Menge an zugeführtem ACH wird in den zweiten Reaktor und gegebenenfalls in weitere Reaktoren eingeführt. Typischerweise erfolgt eine Aufteilung der Gesamtmenge an ACH auf den ersten Reaktor I und der zweiten Reaktor I im Massenverhältnis erster Reaktor I : zweiter Reaktor I im Bereich von 70 : 30 bis 80 : 20, bevorzugt von etwa 75 : 25.

Bevorzugt ist das molare Verhältnis von zugegebener Schwefelsäure zu ACH im ersten Reaktor oder in der ersten Reaktionszone größer als das entsprechende molare Verhältnis in den nachfolgenden Reaktoren oder in den nachfolgenden Reaktionszonen.

In einer besonders bevorzugten Ausführungsform erfolgt die Umsetzung in der zweiten Reaktionsstufe in zwei oder mehreren Schlaufenreaktoren, wobei die Gesamtmenge an ACH in den ersten und mindestens einen weiteren Schlaufenreaktor dosiert wird. Insbesondere bevorzugt umfasst jeder Schlaufenreaktor mindestens eine Pumpe, einen mit Wasser als Medium gekühlten Wärmetauscher, eine Gastrennvorrichtung, mindestens eine Abgasleitung, verbunden mit der Gastrennvorrichtung, und mindestens eine Zuführleitung für ACH in flüssiger Form. Bevorzugt sind die mindestens zwei Schlaufenreaktoren derart miteinander verschaltet, dass die gesamte resultierende Reaktionsmischung des ersten Schlaufenreaktors in die nachfolgenden Reaktoren geführt wird und die Reaktionsmischung in den nachfolgenden Reaktoren mit weiterem flüssigen ACH und optional weiteren Schwefelsäuremengen versetzt wird.

Der erste Schlaufenreaktor wird typischerweise bei einem Kreislaufverhältnis (Verhältnis aus Umwälzvolumenstrom zu Zulaufvolumenstrom) im Bereich von 5 bis 110, bevorzugt bei 10 bis 90, besonders bevorzugt bei 10 bis 70 betrieben. In einem nachfolgenden Schlaufenreaktor liegt das Kreislaufverhältnis vorzugsweise in einem Bereich von 5 bis 100, bevorzugt von 10 bis 90, besonders bevorzugt von 10 bis 70.

Typischerweise wird nach der zweiten Reaktionsstufe (Amidierung) eine zweite Reaktionsmischung erhalten, welche 5 bis 25 Gew.-% Sulfoxyisobuttersäureamid (SIBA), 5 bis 25 Gew.-% Methacrylamid (MASA) sowie < 3 % Hydroxyisobuttersäureamid (HIBA), jeweils bezogen auf die gesamte Reaktionsmischung, gelöst in schwefelsaurer Reaktionsmatrix, enthält.

### Dritte Reaktionsstufe (Konvertierung)

Das erfindungsgemäße Verfahren umfasst in Schritt d. das Konvertieren der zweiten Reaktionsmischung, umfassend das Erhitzen auf eine Konvertierungs-Temperatur im Bereich von 130 bis 200 °C, bevorzugt 130 bis 180 °C, weiter bevorzugt 130 bis 170 °C, insbesondere 140 bis 170 °C, in einem oder mehreren Reaktoren II in einer dritten Reaktionsstufe (Konvertierung), wobei eine dritte Reaktionsmischung, enthaltend überwiegend Methacrylamid (MASA) und Schwefelsäure, erhalten wird.

Typischerweise wird beim Erhitzen der zweiten Reaktionsmischung (Konvertierung), welche eine schwefelsaure Lösung enthaltend SIBA, HIBA und MASA, überwiegend jeweils in Form der Hydrogensulfate, darstellt, auf die Konvertierungs-Temperatur im Bereich von 130 bis 200 °C, bevorzugt 130 bis 180 °C, die Menge an MASA bzw. MASA·H₂SO₄ durch Dehydratisierung des HIBA bzw. SIBA erhöht.

Insbesondere bevorzugt erfolgt die Konvertierung in der dritten Reaktionsstufe bei einer Temperatur im Bereich von 130 bis 200 °C, bevorzugt von 130 bis 180 °C, besonders bevorzugt 140 bis 170 °C und einer Verweilzeit im Bereich von 2 bis 30 Minuten, bevorzugt 3 bis 20 Minuten, insbesondere bevorzugt 5 bis 20 Minuten. Bevorzugt erfolgt das Erhitzen in der dritten Reaktionsstufe (Konvertierung) über einen möglichst kurzen Zeitraum. Insbesondere erfolgt das Erhitzen in der dritten Reaktionsstufe für einen Zeitraum von 1 bis 30 Minuten, bevorzugt 1 bis 20 Minuten, 2 bis 15 Minuten, besonders bevorzugt 2 bis 10 Minuten. In einer bevorzugten Ausführungsform umfasst die dritte Reaktionsstufe (Konvertierung) das Erhitzen der Reaktionsmischung, beispielsweise in einem oder mehreren Vorheizer-Segment(en), und das Führen der Reaktionsmischung bei annähernd adiabatischen Bedingungen, beispielsweise in einem oder mehreren Verweilzeitsegmenten.

Die Konvertierung kann in bekannten Reaktoren durchgeführt werden, welche die Erzielung der genannten Temperaturen in den genannten Zeiträumen ermöglichen. Die Energiezufuhr kann hierbei in bekannter Weise, beispielsweise mittels Wasserdampfs, elektrischer Energie oder elektromagnetischer Strahlung, wie Mikrowellenstrahlung, erfolgen. Bevorzugt wird die Konvertierung in der dritten Reaktionsstufe in einem oder mehreren Wärmetauschern durchgeführt.

In einer bevorzugten Ausführungsform wird die Konvertierung in der dritten Reaktionsstufe in einem Wärmetauscher, umfassend eine zweistufige oder mehrstufige Anordnung von Rohrwendeln, durchgeführt. Vorzugsweise liegen die mehrstufigen Rohrwendeln in einer gegenläufigen Anordnung vor.

Der Wärmetauscher kann beispielsweise mit einem oder mehreren Gasabscheidern kombiniert werden. So ist es beispielsweise möglich, die Reaktionsmischung nach Verlassen der ersten Rohrwendel des Wärmetauschers und / oder nach Verlassen der zweiten Rohrwendel des Wärmetauschers durch einen Gasabscheider zu führen. Dabei können insbesondere gasförmige Nebenprodukte aus der Reaktionsmischung abgetrennt werden.

Typischerweise wird die zweite Reaktionsmischung, erhalten in der zweiten Reaktionsstufe, vollständig in den Reaktor II der dritten Reaktionsstufe geführt.

Bevorzugt wird die dritte Reaktionsmischung, welche nach der Konvertierung erhalten wird, in einen Gasabscheider geführt, wobei gasförmige Nebenprodukte zumindest teilweise von der dritten Reaktionsmischung abgetrennt werden können. Typischerweise wird die entgaste dritte Reaktionsmischung vollständig in die vierte Reaktionsstufe (Veresterung) geführt. Bevorzugt wird das Abgas, welches im Gasabscheider nach der Konvertierung erhalten wird, vollständig oder teilweise aus dem Verfahren ausgeschleust. Weiterhin bevorzugt wird das Abgas, welches im Gasabscheider nach der Konvertierung erhalten wird, vollständig oder teilweise in die vierte Reaktionsstufe (Veresterung) geführt werden.

Insbesondere ermöglicht es das erfindungsgemäße Verfahren, die Menge an störenden Nebenprodukten, bevorzugt die Mengen an MAN, Aceton, MAS und / oder HIBA in der dritten Reaktionsmischung (nach Amidierung und Konvertierung) zu reduzieren. Bevorzugt enthält die dritte Reaktionsmischung kleiner oder gleich 3 Gew.-%, bevorzugt kleiner oder gleich 2 Gew.-% MAS, kleiner oder gleich 2 Gew.-%, bevorzugt kleiner oder gleich 1,5 Gew.-%, weiter bevorzugt kleiner oder gleich 1 Gew.-%, HIBA und kleiner oder gleich 0,3 Gew.-%, insbesondere kleiner oder gleich 0,03 Gew.-%, Methacrylnitril (MAN), jeweils bezogen auf die gesamte dritte Reaktionsmischung.

Bevorzugt enthält die dritte Reaktionsmischung (nach Amidierung und Konvertierung) 30 bis 40 Gew.-% Methacrylamid (MASA), bezogen auf die gesamte dritte Reaktionsmischung. Bevorzugt enthält die dritte Reaktionsmischung (nach Amidierung und Konvertierung) 30 bis 40 Gew.-% MASA, 0 bis 3 Gew.-% MAS und 0,2 bis 1,5 Gew.-%, bevorzugt 0,2 bis 1 Gew.-%, HIBA und 0,001 bis 0,3 Gew.-% MAN, jeweils bezogen auf die gesamte dritte Reaktionsmischung.

### Vierte Reaktionsstufe (Hydrolyse oder Veresterung)

In einer Ausführungsform umfasst das erfindungsgemäße Verfahren in Schritt e. die Umsetzung der dritten Reaktionsmischung, enthaltend überwiegend Methacrylamid, mit Wasser in einem oder mehreren Reaktoren III in einer vierten Reaktionsstufe (Hydrolyse), wobei eine vierte Reaktionsmischung, enthaltend Methacrylsäure, erhalten wird. Unter dem Begriff überwiegend wird insbesondere verstanden, dass der Gehalt der genannten Komponente, bezogen auf die Mischung, mehr als 50 Gew-% beträgt.

Die Hydrolyse, die teilweise auch als Verseifung bezeichnet wird, kann in einem oder mehreren geeigneten Reaktoren III, beispielsweise in beheizten Kesseln oder Rohrreaktoren bei Temperaturen im Bereich von 90 bis 130 °C, bevorzugt von 100 bis 125 °C, durchgeführt werden. Bevorzugt erfolgt die Umsetzung der dritten Reaktionsmischung mit einem Überschuss an Wasser, wobei ein molares Verhältnis von Wasser zu Methacrylamid im Bereich von 1 bis 6, bevorzugt 3 bis 6, in der vierten Reaktionsstufe eingesetzt wird.

Die Hydrolyse mit Wasser liefert typischerweise eine vierte Reaktionsmischung enthaltend Methacrylsäure und gegebenenfalls Hydroxyisobuttersäure (HIBS) und weitere oben beschriebene Nebenprodukte sowie insbesondere Wasser.

In einer weiteren Ausführungsform umfasst das erfindungsgemäße Verfahren in Schritt e. die Umsetzung der dritten Reaktionsmischung, enthaltend überwiegend Methacrylamid, mit Alkohol und Wasser, bevorzugt mit Methanol und Wasser, in einem oder mehreren Reaktoren III in einer vierten Reaktionsstufe (Veresterung), wobei eine vierte Reaktionsmischung, enthaltend Alkylmethacrylat, erhalten wird.

Die Bedingungen der Veresterung im großtechnischen Maßstab sind dem Fachmann bekannt und beispielsweise in US 5,393,918 beschrieben.

Die Umsetzung in der vierten Reaktionsstufe (Veresterung) wird bevorzugt in einem oder mehreren geeigneten Reaktoren III, beispielsweise in beheizten Kesseln durchgeführt. Insbesondere können mit Wasserdampf beheizte Kessel verwendet werden. In einer bevorzugten Ausführungsform erfolgt die Veresterung in zwei oder mehreren, beispielsweise drei oder vier, aufeinander folgenden Kesseln (Kesselkaskade).

Typischerweise wird die Veresterung bei Temperaturen im Bereich von 90 bis 180 °C, bevorzugt von 100 bis 150 °C, bei Drücken von bis zu 7 bar, bevorzugt von kleiner oder gleich 2 bar, und unter Verwendung von Schwefelsäure als Katalysator durchgeführt.

Bevorzugt erfolgt die Umsetzung der dritten Reaktionsmischung mit einem Überschuss an Alkohol und Wasser, bevorzugt einem Überschuss an Methanol und Wasser. Die Zugabe der dritten Reaktionsmischung, enthaltend überwiegend Methacrylamid, und die Zugabe von Alkohol erfolgen bevorzugt in der Art, dass sich ein molares Verhältnis von Methacrylamid zu Alkohol im Bereich von 1:0,7 bis 1:1,6 ergibt. In einer bevorzugten Ausführungsform erfolgt die Umsetzung in der vierten Reaktionsstufe in zwei oder mehreren Reaktoren III, wobei sich im ersten Reaktor III ein molares Verhältnis von Methacrylamid zu Alkohol im Bereich von 1:0,7 bis 1:1,4, bevorzugt im Bereich von 1:0,9 bis 1:1,3, ergibt, und wobei sich im zweiten und den möglichen nachfolgenden Reaktoren III ein molares Verhältnis von Methacrylamid zu Alkohol im Bereich von 1:1,0 bis 1:1,3 ergibt. Generell ist zu beachten, dass gute, effiziente und hochselektive Umsetzungen insbesondere dann erzielt werden, wenn MASA in jedem partiellen Umsetzungsbereich molar in ausreichender Menge Methanol und Wasser zur Verfügung gestellt werden. Das gilt in Summe über alle Reaktoren wie auch für die einzelnen Reaktoren.

Bevorzugt setzt sich der in die vierte Reaktionsstufe (Veresterung) zugeführte Alkohol zusammen aus frisch ins Verfahren zugeführtem Alkohol (Frischalkohol) und aus Alkohol, welcher in zurückgeführten Strömen (Recyclingströmen) des erfindungsgemäßen Verfahrens enthalten ist. Es ist zudem möglich, Alkohol im erfindungsgemäßen Verfahren zu verwenden, welcher in Recyclingströmen aus nachgeschalteten Downstream-Prozessen enthalten ist.

Der Alkohol kann insbesondere ausgewählt sein aus linearen, verzweigten, gesättigten und ungesättigten C₁-C₆-Alkoholen, bevorzugt C₁-C₄-Alkoholen. Insbesondere handelt es sich bei dem Alkohol um einen gesättigten C₁-C₄-Alkohol. Bevorzugt ist der Alkohol ausgewählt aus Methanol, Ethanol, Propanol und Butanol oder verzweigten Isomeren der C3- bis C4-Alkohole. Besonders bevorzugt handelt es sich bei dem Alkohol um Methanol.

Typischerweise erfolgt die Zugabe an Wasser in den Reaktor III oder in die Reaktoren III der vierten Reaktionsstufe derart, dass die Konzentration an Wasser im Bereich von 10 bis 30 Gew.-%, bevorzugt 15 bis 25 Gew.-%, bezogen jeweils auf die gesamte Reaktionsmischung im Reaktor III, liegt.

Im Falle der Hydrolyse von MASA zu MAS, wenn die organische Säure das Zielprodukt darstellt, werden auch höhere Wasserkonzentrationen im Reaktionsprodukt erreicht.

Grundsätzlich kann das in die vierte Reaktionsstufe (Veresterung) zugeführte Wasser aus einer beliebigen Quelle stammen und verschiedene organische Verbindungen enthalten, sofern keine Verbindungen enthalten sind, welche die Veresterung oder die nachfolgenden Verfahrensstufen nachteilig beeinflussen. Bevorzugt stammt das in die vierte Reaktionsstufe zugeführte Wasser aus zurückgeführten Strömen (Recyclingströmen) des erfindungsgemäßen Verfahrens, beispielsweise aus der Aufreinigung des Alkylmethacrylats. Es ist zudem möglich, Frischwasser, insbesondere vollentsalztes Wasser oder Brunnenwasser, in die vierte Reaktionsstufe (Veresterung) zuzuführen.

Die Veresterung mit Methanol liefert typischerweise eine vierte Reaktionsmischung enthaltend Alkylmethacrylat (insbesondere MMA), Hydroxyisobuttersäuremethylester (HIBSM) und weitere oben beschriebene Nebenprodukte sowie signifikante Mengen Wasser und nicht-umgesetzten Alkohol (insbesondere Methanol).

In einer bevorzugten Ausführungsform erfolgt die Veresterung in zwei oder mehreren (insbesondere drei oder vier) aufeinander folgenden Kesseln (Kesselkaskade), wobei der flüssige Überlauf und die gasförmigen Produkte aus dem ersten Kessel in den zweiten Kessel geführt werden. Typischerweise wird mit möglichen nachfolgenden Kesseln in entsprechender Weise verfahren. Insbesondere kann durch eine solche Fahrweise die Schaumbildung in den Kesseln reduziert werden. Im zweiten Kessel und in den möglichen nachfolgenden Kesseln kann ebenfalls eine Zugabe von Alkohol erfolgen. Bevorzugt ist hierbei die Menge an zugegebenem Alkohol um mindestens 10 % kleiner im Vergleich zum vorhergehenden Kessel. Die Konzentrationen an Wasser in den verschiedenen Kesseln können sich typischerweise voneinander unterscheiden. Typischerweise beträgt die Temperatur der in den ersten Kessel eingespeisten dritten Reaktionsmischung im Bereich von 80 bis 180 °C. Typischerweise liegt die Temperatur im ersten Kessel im Bereich von 90 bis 180 °C und die Temperatur im zweiten und in den möglichen nachfolgenden Kesseln im Bereich von 100 bis 150°C, besonders bevorzugt zwischen 100 bis 130°C.

In einer bevorzugten Ausführungsform wird die dritte Reaktionsmischung, welche in der vierten Reaktionsstufe erhalten wird, in gasförmiger Form (Brüden) aus den Reaktoren III abgeführt und der weiteren Aufarbeitung, beispielsweise einem Destillationsschritt, zugeführt. Insbesondere kann die dritte Reaktionsmischung in Form eines Brüdens in den Sumpf einer nachfolgenden Destillationskolonne K1 (Rohkolonne K1) geführt werden. Wenn eine Kaskade aus mehreren Reaktoren III, z.B. mehreren Kesseln, verwendet wird, kann in jedem Kessel die entstehende Reaktionsmischung als Brüdenstrom abgeführt und in die weitere Aufarbeitung geführt werden. Bevorzugt wird lediglich die im letzten Kessel entstehende Reaktionsmischung (als dritte Reaktionsmischung) als Brüdenstrom abgeführt und in die weitere Aufarbeitung geführt. Dieser bei der Veresterung entstehende Brüdenstrom (dritte Reaktionsmischung) stellt typischerweise eine azeotrope Mischung (de facto eine Mischung aus Reinstoffen und diversen Azeotropen) dar, enthaltend Wasser, Alkylmethacrylat, Alkohol, Anteile MAS sowie die beschriebenen Nebenprodukte, z.B. Methacrylnitril und Aceton. Typischerweise weist dieser bei der Veresterung entstehende Brüdenstrom (dritte Reaktionsmischung) eine Temperatur im Bereich von 60 bis 120 °C auf, wobei die Temperatur vom eingesetzten Alkohol abhängt. Typischerweise weist dieser bei der Veresterung entstehende Brüdenstrom eine Temperatur im Bereich von 70 bis 90 °C auf, falls Methanol als Alkohol eingesetzt wird.

Vorteilhaft können ein oder mehrere Stabilisatoren in verschiedenen Stoffströmen des erfindungsgemäßen Verfahrens zugesetzt werden, um eine Polymerisation der Methacrylsäure und / oder des Alkylmethacrylat zu verhindern oder zu reduzieren. Beispielsweise kann ein Stabilisator zu der dritten Reaktionsmischung erhalten nach der Hydrolyse oder Veresterung, zugegeben werden. Weiterhin vorteilhaft kann ein Stabilisator der Kopf-Fraktion des ersten Destillationsschritts K1 (Rohkolonne K1) zugegeben werden. Bewährt hat sich eine Kombination verschiedener Stabilisatoren sowie die Zuführung geringer Mengen sauerstoffhaltiger Gase in die verschiedenen Aufarbeitungsstufen.

Aus der vierten Reaktionsstufe (Veresterung) wird bevorzugt ein Abfallstrom (z.B. (11)) bestehend im Wesentlichen aus verdünnter Schwefelsäure, abgeführt. Dieser Abfallstrom wird typischerweise aus dem Verfahren ausgeschleust. Bevorzugt wird dieser Abfallstrom, insbesondere zusammen mit einem oder mehreren wässrigen Abfallströmen des erfindungsgemäßen Verfahrens, einem Verfahren zur Regenerierung von Schwefelsäure oder einem Verfahren zur Gewinnung von Ammoniumsulfat zugeführt.

### Aufarbeitung der dritten Reaktionsmischung

Das erfindungsgemäße Verfahren umfasst in Schritt f. gegebenenfalls die Abtrennung von Methacrylsäure bzw. Alkylmethacrylat aus der dritten Reaktionsmischung.

Die Abtrennung von Methacrylsäure kann auf jede dem Fachmann bekannte Art realisiert sein und beispielsweise Schritte der Phasentrennung, Extraktion und Destillation umfassen. Ein beispielhaftes Aufarbeitungsverfahren ist in der DE 10 2008 000 787 A1 dargelegt, jedoch können auch andere Sequenzen von Grundoperationen für die Herstellung von Methacrylsäure einer gewünschten Reinheit herangezogen werden.

Die Abtrennung (Aufarbeitung) von Alkylmethacrylat aus der dritten Reaktionsmischung umfasst bevorzugt mindestens zwei Destillationsschritte, bei denen die Nebenprodukte Methacrylnitril (MAN) und Aceton zumindest teilweise in der Kopf-Fraktion als wasserhaltiges Heteroazeotrop erhalten werden und insbesondere dabei vom Alkylmethacrylat zumindest teilweise abgetrennt werden, wobei das wasserhaltige Heteroazeotrop, enthaltend Methacrylnitril (MAN) und Aceton, aus mindestens einem dieser Destillationsschritte zumindest teilweise aus dem Verfahren ausgeschleust wird, und wobei mindestens ein Strom, enthaltend Methacrylnitril und Aceton, zumindest teilweise in die vierte Reaktionsstufe zurückgeführt wird.

Bevorzugt handelt es sich bei dem mindestens einen Strom, enthaltend Methacrylnitril und Aceton, welcher zumindest teilweise in die vierte Reaktionsstufe (Veresterung) zurückgeführt wird, um ein wasserhaltiges Stoffgemisch , enthaltend Methacrylnitril und Aceton, aus mindestens einem der Destillationsschritte wie oben beschrieben.

Beispielsweise können die wässrige Phase und / oder die organische Phase des wasserhaltigen Heteroazeotrops aus mindestens einem Destillationsschritt und / oder Mischungen hiervon aus dem Verfahren ausgeschleust werden, optional nach weiteren Aufarbeitungsschritten, wie Kondensation, Phasentrennung, Extraktion und Waschschritten.

Bevorzugt wird mindestens eine wässrige Phase, welche mittels Kondensation und Phasentrennung des wasserhaltigen Heteroazeotrops aus mindestens einem der Destillationsschritte erhalten wird, vollständig oder teilweise, optional nach einem Extraktionsschritt, in die vierte Reaktionsstufe (Veresterung) zurückgeführt und dort mit der dritten Reaktionsmischung, enthaltend überwiegend Methacrylamid und Schwefelsäure, in Kontakt gebracht.

Bevorzugt wird mindestens eine wässrige Phase, welche mittels Kondensation und Phasentrennung des wasserhaltigen Heteroazeotrops aus mindestens einem der Destillationsschritte erhalten wird, vollständig oder teilweise, optional nach einem Extraktionsschritt, aus dem Verfahren ausgeschleust.

In einer weiteren bevorzugten Ausführungsform wird das wasserhaltige Heteroazeotrops aus mindestens einem der Destillationsschritte zumindest teilweise, optional nach einem Waschschritt, in Form eines gasförmigen Stroms vollständig oder teilweise aus dem Verfahren ausgeschleust. Beispielsweise kann das wasserhaltige Heteroazeotrop aus mindestens einem Destillationsschritt in Form eines Brüdenstroms abgeführt werden und optional nach weiteren Verarbeitungsschritten, beispielsweise ausgewählt aus Kondensation, Phasentrennung, Extraktion und Waschschritten, in gasförmiger Form (als Abgas-Strom) aus dem Verfahren ausgeschleust werden.

Bevorzug umfasst die Abtrennung von Alkylmethacrylat aus der vierten Reaktionsmischung (Schritt f) mindestens einen Phasentrennungsschritt, bei dem das wasserhaltige Heteroazeotrop aus mindestens einem der Destillationsschritte in eine wässrige Phase, enthaltend Methacrylnitril und Aceton, und eine organische Phase, enthaltend überwiegend Alkylmethacrylat, getrennt wird, wobei die wässrige Phase teilweise aus dem Verfahren ausgeschleust wird und / oder teilweise in die vierte Reaktionsstufe zurückgeführt wird, und wobei die organische Phase, enthaltend überwiegend Alkylmethacrylat, vollständig oder teilweise in den mindestens einen Destillationsschritt zurückgeführt wird.

Neben den störenden Nebenprodukten MAN und Aceton enthält das wasserhaltige Heteroazeotrop, welches als Kopf-Fraktion in dem mindestens einen Destillationsschritt erhalten wird, typischerweise Alkohol, beispielsweise Methanol, Wasser und Methylformiat.

In der Regel bildet MAN sowohl mit Methanol als auch mit Methylmethacrylat (MMA) ein Azeotrop, wodurch die Abtrennung von MAN einen hohen Trennaufwand erfordert. Typischerweise wird das störende Nebenprodukt MAN in dem mindestens einen Destillationsschritt wie oben beschrieben daher sowohl in der Kopf-Fraktion als wasserhaltiges Heteroazeotrop als auch in der Sumpf-Fraktion erhalten.

### Rohkolonne (K1) und Vorreinigung

Bevorzugt umfasst die Abtrennung von Alkylmethacrylat in Schritt f des erfindungsgemäßen Verfahrens die Vorreinigung der vierten Reaktionsmischung, welche in der Veresterung erhalten wird. Insbesondere umfasst die Vorreinigung mindestens einen Destillationsschritt K1 (Rohkolonne), mindestens einen Phasentrennungsschritt (z.B. Phasentrenner I) und mindestens einen Extraktionsschritt (z.B. Extraktionsschritt). In einer weiteren Ausführungsform umfasst die Vorreinigung mindestens zwei Destillationsschritte, z.B. Rohkolonne K1 und Roh-Stripper-Kolonne K4, und mindestens einen Phasentrennungsschritt (z.B. Phasentrenner).

Bevorzugt wird die vierte Reaktionsmischung erhalten in der vierten Reaktionsstufe kontinuierlich verdampft, wobei der hierbei entstehenden Brüdenstrom einem ersten Destillationsschritt K1 (Rohkolonne K1) zugeführt wird, bei dem eine Kopf-Fraktion, enthaltend Alkylmethacrylat, Wasser und Alkohol, und eine Sumpf-Fraktion, enthaltend schwerer siedende Komponenten, erhalten werden, und wobei die Sumpf-Fraktion vollständig oder teilweise in die vierte Reaktionsstufe zurückgeführt wird. Insbesondere stellt die Kopf-Fraktion des Destillationsschritts K1 ein wasserhaltiges Heteroazeotrop, enthaltend Methacrylnitril und Aceton, dar. Wasserhaltiges Heteroazeotrop bezeichnet in diesem Fall einen Brüdenstrom, der nach Kondensation und Verflüssigung in zwei Phasen zerfällt, bzw. nach Zugabe von Wasser in unterschiedliche Phasen zerfällt.

In einer bevorzugten Ausführungsform (Variante A) wird die Kopf-Fraktion des Destillationsschritts K1, enthaltend Alkylmethacrylat, Wasser und Alkohol, in einem Phasentrennungsschritt (Phasentrenner I) in eine organische Phase OP-1, enthaltend den überwiegenden Teil des Alkylmethacrylats, und in eine wässrige Phase WP-1, enthaltend Alkohol und weitere wasserlösliche Verbindungen, getrennt, wobei typischerweise die wässrige Phase vollständig oder teilweise in die vierte Reaktionsstufe zurückgeführt wird. Weiterhin bevorzugt wird die organische Phase OP-1, enthaltend den überwiegenden Teil des Alkylmethacrylats, einer Extraktion, bevorzugt unter Verwendung von Wasser als Extraktionsmittel, unterzogen, wobei typischerweise die wässrige Phase dieser Extraktion vollständig oder teilweise in die vierte Reaktionsstufe (Veresterung) zurückgeführt wird.

In einer weiteren bevorzugten Ausführungsform (Variante B) wird die Kopf-Fraktion des Destillationsschritts K1, enthaltend Alkylmethacrylat, Wasser und Alkohol, als Brüdenstrom in einen weiteren Destillationsschritt K4 (z.B. Roh-Stripper-Kolonne (I)) geführt, bei dem ein wasserhaltiges Heteroazeotrop, enthaltend Methacrylnitril und Aceton, als Kopf-Fraktion und eine Sumpf-Fraktion, enthaltend Alkylmethacrylat, erhalten werden. Bevorzugt wird die Kopf-Fraktion des Destillationsschritts K4, optional nach einem Waschschritt, bevorzugt nach einem Waschschritt mit Alkohol (z.B. Methanol), in Form eines gasförmigen Stroms vollständig oder teilweise aus dem Verfahren ausgeschleust. Bevorzugt wird die Sumpf-Fraktion des Destillationsschritts K4 in einem Phasentrennungsschritt (Phasentrenner II) in eine wässrige Phase WP-2, enthaltend Methacrylnitril und Aceton, und eine organische Phase OP-2, enthaltend den überwiegenden Teil des Alkylmethacrylats, getrennt. Typischerweise wird die wässrige Phase WP-2, enthaltend Methacrylnitril und Aceton, vollständig oder teilweise in die vierte Reaktionsstufe (Veresterung) zurückgeführt.

### Azeotrop-Kolonne (K2) und Reinkolonne (K3)

Bevorzugt umfasst die Abtrennung von Alkylmethacrylat aus der vierten Reaktionsmischung (Schritt f), dass eine organische Phase (aus Extraktion oder aus Phasentrenner), enthaltend den überwiegenden Teil des Alkylmethacrylats, in einen Destillationsschritt K2 (Azeotrop-Kolonne) geführt wird, bei dem als Kopf-Fraktion ein wasserhaltiges Heteroazeotrop, enthaltend Methacrylnitril und Aceton, und als Sumpf-Fraktion ein Alkylmethacrylat-Rohprodukt erhalten werden.

Bevorzugt wird der Destillationsschritts K2 (Azeotrop-Kolonne) im Unterdruck durchgeführt. Bevorzugt wird der organische Zulauf des Destillationsschritts K2 vorgewärmt und auf den Kopf der Destillationskolonne K2 geführt. Typischerweise kann der Kopf der Kolonne mittels eines Verdampfers indirekt mit Niederdruck-Dampf beheizt werden.

Bevorzugt wird am Kopf der Destillationskolonne K2 (Azeotrop-Kolonne) ein wasserhaltiges Heteroazeotrop, enthaltend Alkylmethacrylat (insbesondere MMA), Wasser, Alkohol (insbesondere Methanol), Aceton, Methacrylnitril und weitere Leichtsieder, abgetrennt.

Typischerweise wird in dem Destillationsschritt K2 (Azeotrop-Kolonne, eine Sumpf-Fraktion) erhalten, welche den überwiegenden Anteil des Alkylmethacrylats, insbesondere Methylmethacrylat, enthält, und welche nahezu frei von Leichtsieder ist, aber mit Schwersiedern, beispielsweise Methacrylsäure (MAS) und Hydroxyisobuttersäuremethylester (HIBSM), verunreinigt ist. Bevorzugt enthält das Alkylmethacrylat-Rohprodukt, welches als Sumpf-Fraktion des Destillationsschritts K2 (Azeotrop-Destillation) erhalten wird, mindestens 99,0 Gew.-% Alkylmethacrylat. Bevorzugt weist das Alkylmethacrylat-Rohprodukt, welches als Sumpf-Fraktion des Destillationsschritts K2 (Azeotrop-Destillation) erhalten wird, einen MAN-Gehalt von 20 bis 2000 ppm auf.

In einer bevorzugten Ausführungsform wird die Kopf-Fraktion des Destillationsschritts K2 (Azeotrop-Kolonne) zunächst als Brüdenstrom in einem Kondensator geführt und im Vakuum stufenweise kondensiert. Bevorzugt entsteht in dieser stufenweisen Kondensation in der ersten Stufe (saugseitig des Kondensators) ein zweiphasiges Kondensat I und in der zweiten Stufe (druckseitig des Kondensators) ein weiteres Kondensat II. Bevorzugt wird das bei der stufenweisen Kondensation (insbesondere bei der druckseitigen Kondensation) entstehende Abgas, optional nach einem Waschschritt, aus dem Verfahren ausgeschleust.

In einer bevorzugten Ausführungsform (Variante A) wird das zweiphasige Kondensat I aus der ersten Stufe der Kondensation in einen Phasentrenner geführt und das weitere Kondensat II aus der zweiten Stufe der Kondensation als Extraktionsmittel in einem nachgelagerten Extraktionsschritt verwendet.

In einer anderen bevorzugten Ausführungsform (Variante B) werden die flüssigen Phasen der stufenweisen Kondensation vereinigt und in Form eines flüssigen zweiphasigen Stroms in einen Phasentrenner geführt.

Bevorzugt wird das wasserhaltige Heteroazeotrop, welches als Kopf-Fraktion im Destillationsschritt K2 erhalten wird, typischerweise nach der Kondensation, in mindestens eine organische Phase OP-2, enthaltend Alkylmethacrylat, und mindestens eine wässrige Phase WP-2, enthaltend MAN, Aceton und Methanol, in einem Phasentrenner II getrennt. Bevorzugt werden die wässrige Phase WP-2 und / oder die organische Phase OP-2 vollständig oder teilweise aus dem Verfahren ausgeschleust. Insbesondere wird die wässrige Phase WP-2 vollständig oder teilweise in die vierte Reaktionsstufe (Veresterung) zurückgeführt, typischerweise nach einer Phasentrennung. Insbesondere bevorzugt wird die wässrige Phase WP-2, enthaltend Methacrylnitril (MAN) und Aceton, teilweise aus dem Verfahren ausgeschleust und teilweise in die vierte Reaktionsstufe (Veresterung) zurückgeführt.

Oftmals enthält die wässrige Phase WP-2 10 bis 10.000 ppm MAN, bezogen auf die gesamte wässrige Phase WP-2.

Bevorzugt wird die organische Phase OP-2 des wasserhaltigen Heteroazeotrops, welches als Kopf-Fraktion im Destillationsschritt K2 erhalten wird, vollständig oder teilweise, besonders bevorzugt vollständig, in den Destillationsschritt K2 zurückgeführt, typischerweise nach einer Phasentrennung. Insbesondere enthält die organische Phase OP-2 Alkylmethacrylat und Methacrylnitril (MAN).

Typischerweise ist der überwiegende Anteil an MAN, welches in der Kopf-Fraktion des Destillationsschritts K2 enthalten ist, in der organischen Phase (OP-2) des Heteroazeotrops zu finden. Bevorzugt kann durch die vollständige oder teilweise Rückführung der organischen Phase des Heteroazeotrops (OP-2) in den Destillationsschritt K2 eine Anreicherung der störenden Nebenprodukte, insbesondere MAN, und damit eine effektivere Abtrennung, z.B. über die wässrige Phase des Heteroazeotrops (WP-2) erzielt werden. In einer bevorzugten Ausführungsform ist das Gewichtsverhältnis von MAN in der wässrigen Phase WP-2 zu MAN in der organischen Phase OP-2 größer als 0,01.

In einer bevorzugten Ausführungsform wird das Alkylmethacrylat-Rohprodukt aus Destillationsschritt K2 in einen weiteren Destillationsschritt K3 (Reinkolonne) geführt, bei dem das Alkylmethacrylat von schwerer siedenden Verbindungen getrennt wird, und bei dem als Kopf-Fraktion ein Alkylmethacrylat-Reinprodukt erhalten wird. Bevorzugt enthält das Alkylmethacrylat-Reinprodukt aus Destillationsschritt K3 mindestens 99,9 Gew.-%, bevorzugt mindestens 99,95 Gew.-%, bezogen auf das Alkylmethacrylat-Reinprodukt, Alkylmethacrylat. Bevorzugt weist das Alkylmethacrylat-Reinprodukt aus Destillationsschritt K3 einen Gehalt an Methacrylnitril (MAN) im Bereich von 10 bis 300 ppm, bevorzugt 10 bis 100 ppm, besonders bevorzugt 10 bis 80 ppm, insbesondere bevorzugt 50 bis 80 ppm, bezogen auf das Alkylmethacrylat-Reinprodukt, auf. Bevorzugt weist das Alkylmethacrylat-Reinprodukt einen Gehalt an Aceton von kleiner oder gleich 10 ppm, bevorzugt von kleiner oder gleich 2 ppm, besonders bevorzugt von kleiner oder gleich 1 ppm, bezogen auf das Alkylmethacrylat-Reinprodukt, auf.

In einer bevorzugten Ausführungsform wird im zweiten Destillationsschritt K2 (Azeotrop-Kolonne) als Sumpf-Fraktion ein Alkylmethacrylat-Rohprodukt erhalten, welches bevorzugt mindestens 99,0 Gew.-% Alkylmethacrylat enthält, wobei das Alkylmethacrylat-Rohprodukt in einem weiteren Destillationsschritt K3 (Reinkolonne) gereinigt wird, wobei als Kopf-Fraktion ein Alkylmethacrylat-Reinprodukt erhalten wird, welches einen Gehalt an Methacrylnitril im Bereich von 10 bis 300 ppm, bevorzugt 10 bis 100 ppm, besonders bevorzugt 10 bis 80 ppm, insbesondere bevorzugt 50 bis 80 ppm, bezogen auf das Alkylmethacrylat-Reinprodukt, aufweist.

Bevorzugt wird das Alkylmethacrylat-Rohprodukt aus Destillationsschritt K2 in flüssigsiedender Form in den Destillationsschritt K3 (Reinkolonne) geführt. Bevorzugt erfolgt der Zulauf des Destillationsschritts K3 in der Mitte der Reinkolonne K3. Typischerweise erfolgt der Energieeintrag in die Destillationskolonne K3 mittels eines mit Niederdruck-Dampf beheizten Verdampfers. Bevorzugt wird der Destillationsschritt K3 (Reinkolonne), wie der Destillationsschritt K2, im Unterdruck durchgeführt.

Typischerweise wird der am Kopf der Kolonne K3 vollständig kondensierte Destillatstrom in einen Produktstrom und einen Rücklaufstrom in die Kolonne aufgeteilt. Die Qualität des Alkylmethacrylat-Reinprodukts kann beispielsweise über das Rücklaufverhältnis gesteuert werden. Der Sumpfstrom wird bevorzugt in die Veresterung zurückgeführt.

Typischerweise kann die Sumpf-Fraktion des Destillationsschritts K3 (Reinkolonne) (z.B. (O)) vollständig oder teilweise in die vierte Reaktionsstufe (Veresterung) zurückgeführt werden. Insbesondere kann hierdurch enthaltenes Alkylmethacrylat zurückgewonnen werden.

### Variante A

In einer bevorzugten Ausführungsform der Erfindung (auch als Variante A bezeichnet) umfasst die Abtrennung von Alkylmethacrylat aus der vierten Reaktionsmischung, dass
(i) die vierte Reaktionsmischung erhalten in der vierten Reaktionsstufe (Veresterung) zunächst in einem ersten Destillationsschritt K1 (Rohkolonne) destilliert wird, wobei ein erstes wasserhaltiges Heteroazeotrop, enthaltend Methacrylnitril und Aceton, als Kopf-Fraktion erhalten wird;
(ii) das erste wasserhaltige Heteroazeotrop als Kondensat in einem Phasentrennungsschritt (Phasentrenner I) in eine wässrige Phase WP-1 und eine organische Phase OP-1, enthaltend den überwiegenden Teil des Alkylmethacrylats, getrennt wird;
(iii) die organische Phase OP-1, optional nach einem Extraktionsschritt, in einen zweiten Destillationsschritt K2 (Azeotrop-Kolonne) geführt wird, wobei als Kopf-Fraktion ein zweites wasserhaltiges Heteroazeotrop, enthaltend Methacrylnitril und Aceton, erhalten wird;
(iv) zumindest ein Teil des zweiten wasserhaltigen Heteroazeotrops in einem Phasentrennungsschritt (Phasentrenner II) in eine wässrige Phase WP-2, enthaltend Methacrylnitril und Aceton, und eine organische Phase OP-2 getrennt wird,

wobei die organische Phase OP-2 vollständig oder teilweise in den zweiten Destillationsschritt K2 zurückgeführt wird,
und wobei die wässrige Phase WP-2, enthaltend Methacrylnitril und Aceton, teilweise in die vierte Reaktionsstufe (Veresterung) zurückgeführt wird und teilweise, optional nach einem Extraktionsschritt, aus dem Verfahren ausgeschleust wird.

In einer bevorzugten Ausführungsform (Variante A) wird die wässrige Phase WP-1 vollständig oder teilweise in die vierte Reaktionsstufe (Veresterung) zurückgeführt, und die organische Phase OP-1, enthaltend den überwiegenden Teil des Alkylmethacrylats, wird einer Extraktion unter Verwendung von Wasser als Extraktionsmittel unterzogen wird, wobei die wässrige Phase dieser Extraktion in die vierte Reaktionsstufe zurückgeführt wird und die organische Phase dieser Extraktion in den zweiten Destillationsschritt K2 (Azeotrop-Kolonne) geführt wird.

Bevorzugt kann im Phasentrennungsschritt (Phasentrenner II), bei dem zumindest ein Teil des zweiten wasserhaltigen Heteroazeotrops in eine wässrige Phase WP-2 und eine organische Phase OP-2 getrennt wird, Wasser, typischerweise vollentsalztes Wasser oder Brunnenwasser, zugegeben werden, wodurch typischerweise die Phasentrennung verbessert wird.

In einer bevorzugten Ausführungsform (Variante A) wird ein Teil der wässrige Phase WP-2, enthaltend Methacrylnitril und Aceton, einer Extraktion unterzogen wird, wobei eine wässrige Phase WP-3 und eine organische Phase OP-3 erhalten werden, wobei die wässrige Phase WP-3 vollständig oder teilweise aus dem Verfahren ausgeschleust wird, und wobei die organische Phase OP-3 vollständig oder teilweise in die vierte Reaktionsstufe zurückgeführt wird. Optional kann die organische Phase OP-3 zumindest teilweise aus dem Verfahren ausgeschleust werden. Bevorzugt wird die organische Phase OP-3 zusammen mit der Abfallsäure der Veresterung als Spaltsäure aus dem Verfahren ausgeschleust. Insbesondere kann die wässrige Phase WP-3, z.B. zusammen mit der Abfallsäure der Veresterung, einem nachgeschalteten Verfahren zur Regenerierung von Schwefelsäure oder einem nachgeschalteten Verfahren zur Gewinnung von Ammoniumsulfat zugeführt werden.

Typischerweise erfolgt in der oben beschriebenen Variante A die Ausschleusung von störenden Nebenprodukten, insbesondere MAN und Aceton über ein Teil der wässrigen Phase WP-2, wobei durch einen nachgeschalteten Extraktionsschritt der Verlust an Alkylmethacrylat vermindert werden kann.

Bevorzugt wird die Kopf-Fraktion des Destillationsschritts K2 (zweites wasserhaltiges Heteroazeotrop) zunächst als Brüdenstrom in einen Kondensator geführt und im Vakuum stufenweise kondensiert. Bevorzugt wird hierbei ein zweiphasiges Kondensat I in der ersten Stufe der Kondensation (saugseitig des Kondensators) erhalten, welches in einen Phasentrenner geführt wird. Zudem wird bevorzugt ein weiteres Kondensat II in der zweiten Stufe der Kondensation (druckseitig des Kondensators) erhalten, welches als Extraktionsmittel bei der Extraktion der wässrigen Phase WP-2 oder eines Teils der wässrigen Phase WP-2 verwendet wird.

In einer weiteren Ausführungsform wird ein Teil der wässrige Phase WP-2, enthaltend Methacrylnitril und Aceton, einer Extraktion unterzogen, wobei eine wässrige Phase WP-3 und eine organische Phase OP-3 erhalten werden, wobei die wässrige Phase WP-3 einem weiteren Destillationsschritt K5 unterzogen wird, wobei im Destillationsschritt K5 eine Kopf-Fraktion, enthaltend Methacrylnitril, erhalten wird, welche aus dem Verfahren ausgeschleust wird, und wobei im Destillationsschritt K5 eine Sumpf-Fraktion, enthaltend Wasser, erhalten wird, welche vollständig oder teilweise in die Extraktion zurückgeführt wird, und wobei die organische Phase OP-3 vollständig oder teilweise in die vierte Reaktionsstufe zurückgeführt wird. Typischerweise ist die wässrige Sumpf-Fraktion aus Destillationsschritt K5 weitgehend frei von Methacrylnitril. Typischerweise kann mit Hilfe des weiteren Destillationsschritts K5 der ausgeschleuste Abwasserstrom gereinigt und die Entsorgung des Abfallstroms vereinfacht werden.

### Variante B

In einer bevorzugten Ausführungsform der Erfindung (auch als Variante B bezeichnet) umfasst die Abtrennung von Alkylmethacrylat aus der vierten Reaktionsmischung, dass
(i) die vierte Reaktionsmischung erhalten in der vierten Reaktionsstufe zunächst in einem ersten Destillationsschritt K1 (Rohkolonne) destilliert wird, wobei ein erstes wasserhaltiges Heteroazeotrop, enthaltend Methacrylnitril und Aceton, als Kopf-Fraktion erhalten wird;
(ii) das erste wasserhaltige Heteroazeotrop als Brüdenstrom in einen weiteren Destillationsschritt K4 (Roh-Stripper) geführt wird, bei dem ein weiteres wasserhaltiges Heteroazeotrop, enthaltend Methacrylnitril und Aceton, als Kopf-Fraktion und eine Sumpf-Fraktion, enthaltend Alkylmethacrylat, erhalten werden,
(iii) die Kopf-Fraktion aus Destillationsschritt K4, optional nach einem Waschschritt, in Form eines gasförmigen Stroms vollständig oder teilweise aus dem Verfahren ausgeschleust wird;
(iv) die Sumpf-Fraktion aus Destillationsschritt K4 in einem Phasentrennungsschritt (Phasentrenner II) in eine wässrige Phase WP-2, enthaltend Methacrylnitril und Aceton, und eine organische Phase OP-2 getrennt wird, wobei die wässrige Phase WP-2, enthaltend Methacrylnitril und Aceton, vollständig oder teilweise in die vierte Reaktionsstufe zurückgeführt wird,
(v) die organische Phase WP-2 vollständig oder teilweise in einen zweiten Destillationsschritt K2 (Azeotrop-Kolonne) geführt wird, bei dem als Kopf-Fraktion ein zweites wasserhaltiges Heteroazeotrop, enthaltend Methacrylnitril und Aceton, erhalten wird, welches vollständig oder teilweise kondensiert wird und in den Phasentrennungsschritt (Phasentrenner II) nach (iv) geführt wird.

Typischerweise wird im Destillationsschritt K4 (Roh-Stripper) als Kopf-Fraktion ein leichtsiedendes Gemisch enthaltend Methanol, Aceton, Methacrylsäureester und Wasser, und als Kopf-Fraktion ein azeotrop-siedendes Gemisch enthaltend Alkylmethacrylat und Wasser erhalten.

In einer bevorzugten Ausführungsform (Variante B) wird der Rücklauf im Destillationsschritt K4 (Roh-Stripper) mittels eines Partialkondensators erzeugt, welcher so eingestellt ist, dass die Kopf-Fraktion in Form eines Brüden aus der Kolonne K4 ausgeschleust wird und, ein flüssiges Kondensat enthaltend Alkylmethacrylat in die Kolonne als Rücklauf zurückgefahren wird. Ein Teil des Rücklaufs der Destillationskolonne K4 wird bevorzugt in Form eines flüssigen Seitenstroms abgeführt und als Rücklauf in Destillationsschritt K1 (Roh-Kolonne,) geführt.

Typischerweise ist die Sumpf-Fraktion des Destillationsschritts K4 (Roh-Stripper) ein azeotropes Gemisch enthaltend Alkylmethacrylat, Wasser, geringen Mengen Leichtsieder (z.B. Methanol, Aceton) und Schwersieder (z.B. Hydroxyisobuttersäureester). Bevorzugt wird die Sumpf-Fraktion aus Destillationsschritt K4 abgekühlt und in einem Phasentrenner II, bevorzugt zusammen mit einem weiteren Rücklaufstrom, in eine organische Phase OP-2 und ein wässrige Phase WP-2 aufgetrennt. Typischerweise enthält die wässrige Phase WP-2 Wasser, Alkohol, Aceton und Alkylmethacrylat. Bevorzugt kann die wässrigen Phase WP-2 mit Frischwasser, z.B. vollentsalztes Wasser (VE-Wasser), vermischt werden und in Form eines vereinigten Rücklaufstroms der Veresterung zugeführt werden. Typischerweise kann hierdurch der Wasserbedarf der Veresterung gedeckt werden und Edukte zurückgewonnen werden.

Bevorzugt wird die Kopf-Fraktion aus Destillationsschritt K4 als Brüdenstrom in eine Abgas-Waschkolonne geführt und dort mit frischem Alkohol, z.B. Methanol, als Waschmedium gewaschen. Bevorzugt wird der gewaschene Abgas-Strom vollständig oder teilweise aus dem Verfahren ausgeschleust. Bevorzugt wird im Sumpf der Abgas-Waschkolonne der organische Strom, enthaltend Methanol und Alkylmethacrylat, erhalten, welcher in die Veresterung zurückgeführt wird. Dieser organische Rücklaufstrom kann hierbei auf verschiedene Veresterungsreaktoren verteilt werden.

### Weitere Schritte

In einer bevorzugten Ausführungsform umfasst das erfindungsgemäße Verfahren eine Regenerierung von Schwefelsäure, wobei ein Teil der vierten Reaktionsmischung erhalten in der vierten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt werden, bei dem Schwefelsäure erhalten wird, welche in die vierte Reaktionsstufe zurückgeführt wird.

In einer bevorzugten Ausführungsform umfasst das erfindungsgemäße Verfahren eine Gewinnung von Ammoniumsulfat, wobei ein Teil der vierten Reaktionsmischung erhalten in der vierten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt wird, bei dem mittels Kristallisation Ammoniumsulfat erhalten wird, welches als Nebenprodukt abgetrennt wird.

Bevorzugt wird ein Abfallstrom, bestehend im Wesentlichen aus verdünnter Schwefelsäure, welcher aus dem Reaktor III der Veresterung abgeführt wird, und / oder ein oder mehrere wässrige Abfallströmen des Verfahrens einem Verfahren zur Regenerierung von Schwefelsäure oder einem Verfahren zur Gewinnung von Ammoniumsulfat zugeführt.

Verfahren zur Regenerierung von Schwefelsäure und Verfahren zur Gewinnung von Ammoniumsulfat aus Spaltsäure sind dem Fachmann bekannt und beispielsweise in WO 02/23088 A1 und WO 02/23089 A1 beschrieben. Die Einbettung von Verfahren zur Regenerierung von Schwefelsäure in ein Verfahren zur Herstellung von Alkylmethacrylaten nach dem ACH-Sulfo-Verfahren ist beispielsweise in DE 10 2006 059 513 oder DE 10 2006 058 250 beschrieben.

### Beschreibung der Figuren

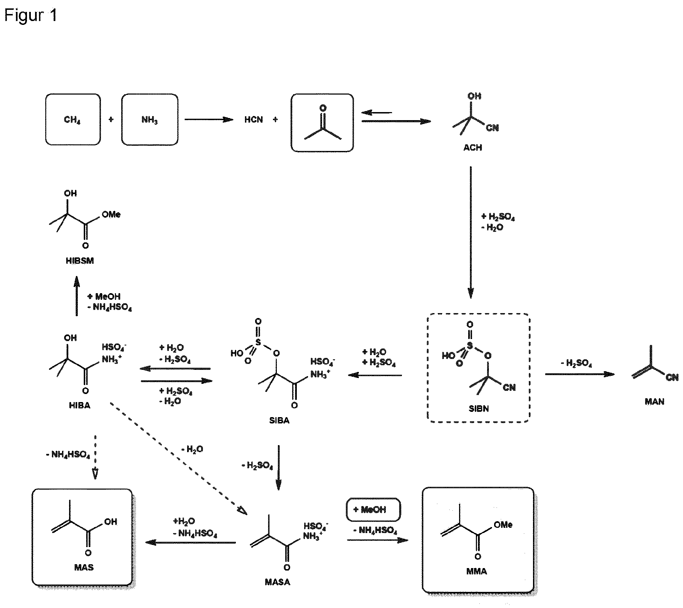

Figur 1 beschreibt das Reaktionsnetzwerk der Bildung von Methacrylsäure und / oder Methylmethacrylat ausgehend von Methan und Ammoniak sowie Aceton. Ausgehend von Methan (CH₄) und Ammoniak (NH₃) kann über das BMA-Verfahren (Blausäure aus Methan und Ammoniak) mittels katalytischer Dehydrierung Blausäure hergestellt werden (CH₄ + NH₃ → HCN + 3 H₂) (Variante 1). Alternativ kann über das Andrussow-Verfahren ausgehend von Methan und Ammoniak, unter Zugabe von Sauerstoff, Blausäure hergestellt werden (CH₄ + NH₃ + 1,5 O₂ → HCN + 3 H₂O) (Variante 2). Im nächsten Schritt wird ausgehend von Aceton und Blausäure, unter Zugabe eines basischen Katalysators (z.B. Diethylamin Et₂NH oder anorganischen Basen) Acetoncyanhydrin (ACH) hergestellt. Die Hydroxygruppe des Acetoncyanhydrins wird im Folgenden mit Schwefelsäure verestert, wobei zunächst Sulfoxyisobutyronitril (SIBN) erhalten wird. Die Nitrilgruppe des Sulfoxyisobutyronitrils (SIBN) kann im nächsten Schritt unter Einwirkung von Schwefelsäure und Wasser verseift werden, wobei Sulfoxyisobuttersäureamid-Hydrogensulfat (SIBA·H₂SO₄) erhalten wird. Als Nebenreaktion kann die Bildung von Methacrylnitril (MAN) unter Eliminierung von Schwefelsäure aus SIBN erfolgen. Sulfoxyisobuttersäureamid-Hydrogensulfat (SIBA·H₂SO₄) kann zudem teilweise zum alpha-Hydroxyisobuttersäureamid-hydrogensulfat (alpha-Hydroxyisobutyramid-hydrogensulfat, HIBA·H₂SO₄) hydrolysiert werden. Ebenfalls ist die Rückreaktion zum Schwefelsäureester SIBA·H₂SO₄ möglich. Als Nebenprodukt kann alpha-Hydroxyisobuttersäure (HIBS) durch weitere Hydrolyse von HIBA·H₂SO₄ gebildet werden. Ausgehend von SIBA·H₂SO₄ wird unter Eliminierung von Schwefelsäure Methacrylamid-hydrogensulfat (MASA·H₂SO₄) gebildet (Konvertierung). Ebenfalls kann die langsame Umsetzung von HIBA oder HIBS zu MAS oder MASA als Eliminierungsreaktion unter Abspaltung von NH₄HSO₄ oder Wasser ablaufen. Nachfolgend kann Methacrylamid-hydrogensulfat (MASA·H₂SO₄) durch Hydrolyse zu Methacrylsäure (MAS) oder durch Veresterung mit Methanol (MeOH) zu Methylmethacrylat (MMA) umgewandelt werden. Wird alpha-Hydroxyisobuttersäure (HIBS) in die Veresterung mitgeschleppt, kann sie zu alpha-Hydroxyisobuttersäuremethylester (HIBSM) umgesetzt werden.

Die Abkürzungen in Figur 1 haben die folgenden Bedeutungen:
- ACH: Acetoncyanhydrin;
- SIBN: alpha-Sulfoxy-isobuttersäurenitril, auch als Sulfoxyisobutyronitril bezeichnet;
- SIBA: alpha-Sulfoxyisobuttersäureamid, auch als Sulfoxyisobuttersäureamid oder Sulfoxyisobutyramid bezeichnet;
- SIBA·H₂SO₄: alpha-Sulfoxyisobuttersäureamid-Hydrogensulfat, auch als Sulfoxyisobuttersäureamid-Hydrogensulfat bezeichnet;
- MAN: Methacrylnitril;
- HIBA: alpha-Hydroxyisobuttersäureamid; alpha-Hydroxyisobutyramid;
- HIBA·H₂SO₄: alpha-Hydroxyisobuttersäureamid-Hydrogensulfat, alpha-Hydroxyisobutyramid-Hydrogensulfat;
- MASA: Methacrylsäureamid / Methacrylamid;
- MASA·H₂SO₄: Methacrylamid-Hydrogensulfat;
- MAS: Methacrylsäure;
- MMA: Methylmethacrylat;
- HIBS: alpha-Hydroxyisobuttersäure

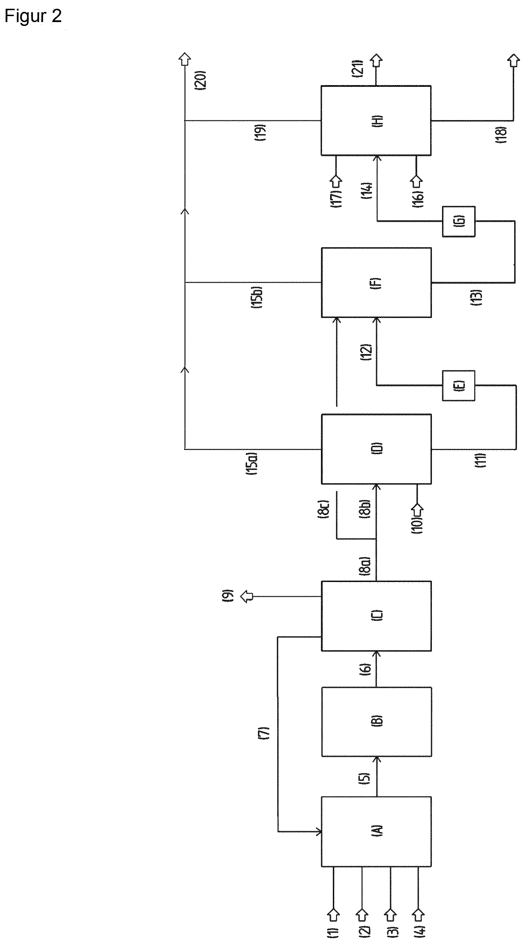

Figur 2 zeigt ein Fließschema einer bevorzugten Ausführungsform der zweiten und dritten Reaktionsstufe (Amidierung und Konvertierung, D bis G) des erfindungsgemäßen Verfahrens sowie der ersten Reaktionsstufe (A, B) und der Aufarbeitung (C) des hergestellten ACH. Es sind zwei aufeinander folgende Verfahrensschritte der Amidierung und Konvertierung umfassend die erste Amidierung (D) und die erste Konvertierung (E) sowie die zweite Amidierung (F) und die zweite Konvertierung (G) dargestellt.

In Figur 2 haben die Bezugszeichen die folgenden Bedeutungen:
Apparate
   - (A): Reaktor 1 ACH-Synthese
   - (B): Reaktor 2 ACH-Synthese
   - (C): ACH-Destillation, Aufarbeitung
   - (D): Amidierungsreaktor (erster Reaktor I)
   - (E): erster Konverter
   - (F): Amidierungsreaktor (zweiter Reaktor I)
   - (G): zweiter Konverter
   - (H): Hydrolyse oder Veresterung
Stoffströme
   - (1): Aceton-Zulauf (Aceton-Reaktant)
   - (2): Blausäure-Zulauf
   - (3): Diethylamin-Zulauf
   - (4): Schwefelsäure-Zulauf
   - (5): Synthesegemisch
   - (6): erste Reaktionsmischung, Roh-ACH zur Destillation
   - (7): Leichtsieder-Recyclestrom
   - (8a): Rein-ACH-Mischung
   - (8b): ACH-Zulauf 1. Stufe, (erster Teilstrom Rein-ACH-Mischung)
   - (8c): ACH-Zulauf 2. Stufe (zweiter Teilstrom Rein-ACH-Mischung)
   - (8): Entgastes Amidgemisch
   - (9): Abgas ACH-Destillation
   - (10): Schwefelsäure-Zulauf
   - (11): Anrührgemisch 1. Stufe
   - (12): Zwischenkonvertiertes Reaktionsgemisch
   - (13): Anrührgemisch 2. Stufe
   - (14): Konvertiertes Reaktionsgemisch (dritte Reaktionsmischung)
   - (15a): Abgas Amidierungsreaktoren Stufe 1
   - (15b): Abgas Amidierungsreaktoren Stufe 2
   - (16): optionaler Methanol-Zulauf
   - (17): Wasser-Zulauf
   - (18): Spaltsäure
   - (19): Abgas Veresterung
   - (20): Gesamtabgas
   - (21): Rein-MAS oder MMA

### Beispiele

Die Herstellung von Methacrylamid in schwefelsaurer Lösung, umfassend die Herstellung von Acetoncyanhydrin in einer ersten Reaktionsstufe, dessen Umsetzung mit Schwefelsäure in der Amidierung der zweiten Reaktionsstufe, die thermische Umsetzung der zweiten Reaktionsmischung in der Konvertierung der dritten Reaktionsstufe sowie die anschließende Veresterung mit Methanol und Wasser in der dritten Reaktionsstufe, erfolgte anhand der Ausführungsform nach Figur 2.

Im Folgenden werden fünf erfindungsgemäße Beispiele (Beispiele 1 bis 5) unter Verwendung variierender Wassergehalte im ACH (8a, 8b, 8c), das in der ersten Reaktionsstufe (A, B) und der Aufarbeitung (C) erhalten und der zweiten Reaktionsstufe (D, F) zugeführt wurde, sowie in der Schwefelsäure (10) im Zulauf zur Amidierung (D), mit vier Vergleichsbeispielen (Beispiele 6 bis 9) verglichen.

Zur Ermittlung der jeweiligen Amidierungsausbeute wurden Proben nach dem zweiten Konvertierungs-Reaktor (G) entnommen. Die nach Quantifizierung mittels HPLC ermittelten Konzentrationen von MASA, MAS und HIBA wurden für die Bilanzierung der Verfahrensschritte der Amidierung (zweite Reaktionsstufe, D, F) und der Konvertierung (dritte Reaktionsstufe, E, G) herangezogen. Ergebnisse bezüglich der einzelnen Verfahrensschritte sind für die Vergleichsbeispiele und die erfindungsgemäßen Beispiele in den Tabellen 1 bis 8 dargestellt. Bei Auflistung der Messergebnisse ist jeweils ein Messfehler der HPLC-Analytik von ± 0.2 % ausgewiesen.

Die aus der Konvertierung (G) erhaltene dritte Reaktionsmischung (14), enthaltend MASA, MAS und HIBA wurde anschließend in einer Kaskade von mehreren Veresterungsreaktoren III (H) mit Methanol und Wasser versetzt und verestert; in einer aufgesetzten Trennkolonne wurde MMA-haltiges Roh-Gemisch als Brüden entnommen, kondensiert (21) und in eine organische und wässrige Phase getrennt. MMA wurde erhalten durch Aufarbeitung der organischen Phase.

Der Wassergehalt in den Schwefelsäure-Zuläufen (4, 10) wurde über Bilanzierung basierend auf dem Schwefelsäuregehalt in den Strömen bestimmt, wobei der Schwefelsäuregehalt durch Messung der Dichte und der Schallgeschwindigkeit ermittelt wurde. Der Wassergehalt des Aceton-Zulaufs (1) wurde gaschromatographisch unter Verwendung eines Wärmeleitfähigkeits-Detektors bestimmt. Der Wassergehalt des Blausäure-Zulaufs (2) wurde durch Karl-Fischer-Titration ermittelt, und der Wassergehalt der Rein-ACH-Mischung (8a) wurde über Bilanzierung basierend auf dem ACH-Gehalt bestimmt, wobei der ACH-Gehalt mittels HPLC ermittelt wurde.

### Erfindungsgemäßes Beispiel 1:

Methacrylamid wurde hergestellt aus Acetoncyanhydrin und Schwefelsäure mit einem Gesamtwassergehalt von 18,48 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

9500 kg/h eines Aceton-Reaktanten (1), mit einem Wassergehalt gemäß Tabelle 10, wurden mit 4439 kg/h (2) Blausäure (HCN), welche einen Wassergehalt von ca. 0,03 Gew.-%, bezogen auf den Gesamtstrom (2), aufwies, sowie 8 kg/h Diethylamin (3) als Katalysator in Reaktor (A) in der Flüssigphase zur Reaktion gebracht. Zusätzlich wurde ein Kreislauf-Destillatstrom (7) der ACH-Destillation (C), welcher ACH, Aceton und HCN enthält, in Reaktor (A) rückgeführt.

Reaktor (A) ist als Schlaufenreaktor mit nachgeschaltetem Verweilzeitbehälter (B) ausgeführt, wobei die freiwerdende Reaktionswärme im Reaktor (A) mittels Kühlwassers über einen Rohrbündelwärmetauscher abgeführt wurde. Die Reaktion wurde bei 35 bis 40 °C und Normaldruck betrieben.

Das im Reaktor (A) erhaltene, ca. 40 °C heiße Synthesegemisch (5), das mehr als 85 Gew.-% ACH, bezogen auf den Gesamtstrom (5), enthielt, wurde kontinuierlich dem nachgeschalteten Verweilzeitbehälter (B) zugeführt, in dem das Synthesegemisch (5) nachreifte. Der Verweilzeitbehälter (B) ist als gekühlter Vorlagebehälter mit Umpumpkreislauf ausgeführt und wurde bei ca. 10 °C und Normaldruck betrieben.

Das nach dem Verweilzeitbehälter (B) erhaltene Roh-ACH (erste Reaktionsmischung, 6), mit einem gesteigerten ACH-Gehalt von 92,7 Gew.-%, bezogen auf den Gesamtstrom (6), wurde anschließend kontinuierlich einem Destillationsschritt (C) zugeführt. Zur Stabilisierung des gewonnenen ACH sowie zur Neutralisation des noch enthaltenen Katalysators Diethylamin wurden dem Roh-ACH (erste Reaktionsmischung, 6) 32 kg/h 98%-ige Schwefelsäure (4) über eine Mischstrecke zugeführt. Es resultierte ein stabilisierter Roh-ACH-Strom (6 a).

Der stabilisierte Roh-ACH-Strom (6a) wurde kontinuierlich am Kopf einer Destillationskolonne (C) zugeführt. Die Destillationskolonne (C) wurde als Abtriebs-Kolonne bei ca. 120 mbar betrieben, indirekt mit Heizdampf (10 bar) beheizt und trennte Aceton, HCN sowie weitere leichtsiedende Nebenprodukte von einer Rein-ACH-Mischung (8a) als Sumpfprodukt, die 98,5 Gew.-% ACH, bezogen auf den Gesamtstrom (8a), und als Nebenprodukte Wasser und Aceton enthielt. Die entsprechend erhaltene Zusammensetzung ist Tabelle 10 zu entnehmen.

Das leichtsiedende Destillat der Destillation (C), wurde zusammen mit einem erhaltenen Vakuumpumpenkondensat als leichtsiedender Recyclestrom (7) kontinuierlich in die erste Reaktionsstufe (A) zurückgeführt.

Die Abluft (9), welche in der Vakuumstation der Destillation (C) anfiel, wurde kontinuierlich aus dem Prozess entfernt und kontrolliert einer Verbrennung zugeführt.

Die gewonnene Rein-ACH-Mischung (8a), mit einem Massenstrom von 13850 kg/h, wurde nach der Destillation (C) auf 10 °C abgekühlt. Anschließend wurde der Gesamtstrom der Rein-ACH-Mischung (8a) auf zwei Teilströme (8b, 8c) aufgeteilt, die in der zweiten Reaktionsstufe in den ersten Reaktor I (D) beziehungsweise den zweiten Reaktor I (F) der Amidierung zugeführt wurden.

Der Teilstrom 8b, mit einem Massenstrom von 9000 kg/h, wurde mit dem Schwefelsäure-Strom (10), der insgesamt einen Massenstrom von 22440 kg/h aufwies, und dessen Zusammensetzung in Tabelle 10 ausgewiesen ist, auf den ersten Amidierungs-Reaktor I (D) aufgegeben. Der Schwefelsäure-Strom 10 wurde vor Eintritt in den ersten Amidierungs-Reaktor I mit 50 ppm Phenothiazin als Stabilisator versetzt.

Der erste Amidierungs-Reaktor I (D) ist als Schlaufenreaktor ausgeführt und wurde bei 98 °C betrieben. Der Teilstrom (8b) wurde kontinuierlich und mit einer Temperatur von 20 °C dem ersten Amidierungs-Reaktor I (D) zugeführt.

Die für die optimale Umsetzung des ACH in dem ersten Reaktor I (D) und dem zweiten Reaktor I (F) notwendige Schwefelsäuremenge (in Strom 10), wurde in einem Massenverhältnis zur Gesamtmenge an ACH im Zulauf (8b+8c) von 1,62 kg_{H2SO4}/kg_{ACH} bzw. 1,41 mol _{H2SO4}/mol_{ACH} in den ersten Reaktor I (D) zugeführt.

Aus dem ersten Reaktor I (D) wurde ein heißes Anrührgemisch (11) mit 98 °C erhalten, das Sulfoxy-isobuttersäureamid (SIBA), Methacrylsäureamid (MASA) sowie Hydroxyisobuttersäureamid (HIBA), jeweils gelöst in Schwefelsäure, enthielt. Das Anrührgemisch (11) wurde nach einer Gasabscheidung kontinuierlich einer Zwischenkonvertierung im ersten Konverter (E) zugeführt. Die zur Förderung benötigte Druckdifferenz wurde durch die Reaktorkreislaufpumpe des Amidierungs-Reaktors (D) realisiert. Das entstehende Abgas (15a) wurde in Richtung Amidierungs-Abluft (20) aus dem Prozess abgeführt.

Der erste Konverter (E) ist als Strömungsrohrreaktor ausgeführt, der ein Vorheizer-Segment und ein Verweilzeit-Segment umfasst. Das in den ersten Konverter (E) eintretende Anrührgemisch (11) wurde auf 130°C im Vorheizer-Segments erhitzt. Anschließend wurde in dem Verweilzeit-Segment nach konvertiert.

Das aus dem ersten Konverter (E) austretende Reaktionsgemisch (12) wurde dann dem zweiten Amidierungs-Reaktor I (F) zugeführt. Der zweite Amidierungs-Reaktor (F) ist analog zum ersten Amidierungs-Reaktor I (D) als Schlaufenreaktor aufgebaut und wurde ebenfalls bei ca. 98 °C betrieben. Der im zweiten Amidierungs-Reaktor I (F) benötigte, ACH enthaltende Teilstrom 8c mit 4850 kg/h wurde auch im zweiten Amididierungs-Reaktor I (F) direkt in die Reaktionsmischung (12) aufgegeben. Entstehendes Abgas (15b) wurde in Richtung Gesamtabgas (20) aus dem Prozess abgeführt.

Zur finalen Umsetzung der reaktiven Bestandteile des Reaktionsgemischs (13), das aus dem zweiten Amidierungs-Reaktor I (F) erhalten wurde, wurde anschließend ein zweiter Konvertierungsschritt im zweiten Konverter (G) durchgeführt.

Der zweite Konverter (G) ist ebenfalls als Strömungsrohrreaktor ausgeführt und umfasst ein Vorheizer-Segment und ein Verweilzeit-Segment.

Das in den zweiten Konverter (G) eintretende Reaktionsgemisch (13) wurde zunächst auf eine optimale Temperatur, welche in Tabelle 10 ausgewiesen ist, im Vorheizer-Segment erhitzt. Die im zweiten Konverter (G) einzustellende Temperatur wurde durch Vorversuche ermittelt und führte zu einem maximalen Umsatz von SIBA und HIBA zu MASA.

Anschließend wurde das Reaktionsgemisch (13) im Verweilzeit-Segment des zweiten Konverters (G) nachkonvertiert, wobei die zuvor eingestellte Temperatur beibehalten wurde.

Das aus dem zweiten Konverter (G) erhaltene Reaktionsgemisch wurde dann von gasförmigen Nebenkomponenten abgetrennt, welche in Form eines Abgasstroms (15c) abgeführt wurden. Die resultierende dritte Reaktionsmischung (14) wurde kontinuierlich dem zweiten Konverter (G) in flüssiger Form entzogen. Die dritte Reaktionsmischung (14) enthielt die Komponenten Methacrylsäureamid (MASA), Methacrylsäure (MAS) sowie Hydroxyisobuttersäureamid (HIBA) gemäß Tabelle 10. Die jeweilige HPLC-Analyse zur Bestimmung der jeweiligen Komponenten wurde dreifach durchgeführt; die jeweiligen arithmetischen Mittelwerte sind in Tabelle 1 eingetragen. Probenahme und Analyse erfolgten zweimal pro Tag, wobei im stationären Betrieb der Anlage insgesamt fünf Tage in Folge Proben genommen wurden.

Die erhaltene Summenausbeute an zum Zielprodukt (Methylmethacrylat) umsetzbaren Komponenten (Methacrylsäureamid, Methacrylsäure), welche anschließend einer Veresterung (H) zur Herstellung von Methylmethacrylat zugeführt wurden, ist ebenfalls in Tabelle 1 dargestellt. Die angegebenen Konzentrationen beziehen sich auf den Gesamtstrom der dritten Reaktionsmischung (14). Die mittlere Ausbeute von 10 repräsentativen Proben, gemessen im stationären Betrieb eines Prozesses mit den genannten Bedingungen, betrug 93,0%. In Strom (14) wurde mittels HPLC ein MAN-Gehalt von 120 ppm gemessen.

| **Tabelle 1: Ergebnisse erfindungsgem. Beispiel 1** | | **Konzentration im Produktstrom [Gew.-%]** | | | **Ausbeute [%]** |
|---|---|---|---|---|---|
| Prob e | Zeit | Methacrylami d | Methacrylsäur e | Hydroxyisobuttersäureami d | Methacrylamid + Methacrylsäure |
| 1 | Tag 1; 10:10 | 33,81 | 1,20 | 1,60 | 93,04 |
| 2 | Tag 1; 13:40 | 33,90 | 1,15 | 1,63 | 93,29 |
| 3 | Tag 2; 07:30 | 33,72 | 1,15 | 1,55 | 92,79 |
| 4 | Tag 2; 10:26 | 33,78 | 1,12 | 1,58 | 92,96 |
| 5 | Tag 3; 09:05 | 33,80 | 1,17 | 1,56 | 93,01 |
| 6 | Tag 3; 11:08 | 33,98 | 1,17 | 1,54 | 93,51 |
| 7 | Tag 4; 10:34 | 34,00 | 1,12 | 1,53 | 93,56 |
| 8 | Tag 4; 14:11 | 33,71 | 1,12 | 1,51 | 92,76 |
| 9 | Tag 5; 07:46 | 33,76 | 1,18 | 1,62 | 92,90 |
| 10 | Tag 5; 09:19 | 33,40 | 1,19 | 1,81 | 91,91 |
| **Mittelwert** | | | | | **93,0** |
| | | | | | Schwankungsbereic h der Ausbeute 1,7% |

### Erfindungsgemäßes Beispiel 2:

Methacrylamid wurde hergestellt aus Acetoncyanhydrin und Schwefelsäure mit einem Gesamtwassergehalt von 3,66 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 2 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 2 zusammengefasst sind. Die sich daraus ergebende Ausbeute ist ebenfalls in Tabellen 2 und 10 dargestellt.

Die mittlere Ausbeute von 10 repräsentativen Proben, gemessen im stationären Betrieb des Prozesses mittels HPLC, betrug unter den genannten Bedingungen 94,5 %.

| **Tabelle 2: Ergebnisse erfindungsgem. Beispiel 2** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäureamid | Methacrylamid + Methacrylsäure |
| 1 | Tag 1; 16:10 | 34,66 | 1,03 | 1,03 | 94,49 |
| 2 | Tag 1; 19:50 | 34,51 | 1,05 | 1,08 | 94,08 |
| 3 | Tag 2; 07:20 | 34,72 | 1,05 | 0,98 | 94,65 |
| 4 | Tag 2; 11:26 | 34,78 | 1,02 | 1,03 | 94,82 |
| 5 | Tag 3; 07:05 | 34,80 | 1,04 | 1,04 | 94,87 |
| 6 | Tag 3; 10:09 | 34,68 | 1,07 | 1,04 | 94,54 |
| 7 | Tag 4; 08:30 | 34,50 | 1,01 | 1,01 | 94,05 |
| 8 | Tag 4; 14:11 | 34,71 | 1,02 | 1,06 | 94,63 |
| 9 | Tag 5; 07:30 | 34,76 | 1,03 | 0,96 | 94,76 |
| 10 | Tag 5; 10:29 | 34,40 | 0,90 | 1,00 | 93,78 |
| **Mittelwert** | | | | | **94,5 %** |
| | | | | | Schwankungsb ereich der Ausbeute 1,1% |

### Erfindungsgemäßes Beispiel 3:

Methacrylamid wurde hergestellt aus Acetoncyanhydrin und Schwefelsäure mit einem Gesamtwassergehalt von 0,47 mol% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 3 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 3 zusammengefasst sind. Die sich daraus ergebende Ausbeute ist ebenfalls in Tabellen 3 und 10 dargestellt.

Die mittlere Ausbeute von 10 repräsentativen Proben, gemessen im stationären Betrieb des Prozesses mittels HPLC, betrug unter den genannten Bedingungen 92,2 %.

| **Tabelle 3: Ergebnisse erfindungsgem. Beispiel 3** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäureamid | Methacrylamid +Methacrylsäure |
| 1 | Tag 1; 09:05 | 35,01 | 1,21 | 0,06 | 92,18 |
| 2 | Tag 1; 10:50 | 34,80 | 0,93 | 0,08 | 91,63 |
| 3 | Tag 2; 07:30 | 35,31 | 0,86 | 0,05 | 92,97 |
| 4 | Tag 2; 10:15 | 34,70 | 0,89 | 0,03 | 91,36 |
| 5 | Tag 3; 07:20 | 35,26 | 0,98 | 0,08 | 92,84 |
| 6 | Tag 3; 10:19 | 34,93 | 0,96 | 0,03 | 91,97 |
| 7 | Tag 4; 08:22 | 35,05 | 0,99 | 0,03 | 92,29 |
| 8 | Tag 4; 11:04 | 35,08 | 0,95 | 0,06 | 92,36 |
| 9 | Tag 5; 07:35 | 34,78 | 1,01 | 0,08 | 91,57 |
| 10 | Tag 5; 10:29 | 35,29 | 0,97 | 0,07 | 92,92 |
| **Mittelwert** | | | | | **92,2** |
| | | | | | %Schwankungs bereich der Ausbeute 1,6% |

### Erfindungsgemäßes Beispiel 4:

Methacrylamid wurde hergestellt aus Acetoncyanhydrin und Schwefelsäure mit einem Gesamtwassergehalt von 15,67 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 4 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 4 zusammengefasst sind. Die sich daraus ergebende Ausbeute ist ebenfalls in Tabellen 4 und 10 dargestellt.

Die mittlere Ausbeute von 10 repräsentativen Proben, gemessen im stationären Betrieb des Prozesses mittels HPLC, betrug unter den genannten Bedingungen 94,1 %.

| **Tabelle 4: Ergebnisse erfindungsgem. Beispiel 4** | | **Konzentration im Produktstrom [Gew.-%]** | | | **Ausbeute [%]** |
|---|---|---|---|---|---|
| Pro be | Zeit | Methacrylamid | Methacrylsäure | Hydroxyisobuttersäurea mid | Methacrylamid + Methacrylsäure |
| 1 | Tag 1; 10:05 | 34,49 | 1,13 | 1,12 | 94,49 |
| 2 | Tag 1; 12:50 | 34,30 | 1,15 | 1,11 | 93,97 |
| 3 | Tag 2; 08:30 | 34,32 | 1,05 | 1,06 | 94,02 |
| 4 | Tag 2; 11:30 | 34,33 | 1,13 | 1,08 | 94,05 |
| 5 | Tag 3; 07:10 | 34,31 | 1,10 | 1,12 | 94,00 |
| 6 | Tag 3; 10:20 | 34,38 | 1,07 | 1,11 | 94,19 |
| 7 | Tag 4; 08:20 | 34,28 | 1,11 | 1,06 | 93,91 |
| 8 | Tag 4; 11:22 | 34,28 | 1,12 | 1,11 | 93,91 |
| 9 | Tag 5; 07:30 | 34,29 | 1,17 | 1,12 | 93,94 |
| 10 | Tag 5; 10:20 | 34,30 | 1,06 | 1,09 | 93,97 |
| **Mittelwert** | | | | | **94,1 %** |
| | | | | | Schwankungsb reite der Ausbeute 0,6% |

### Erfindungsgemäßes Beispiel 5:

Methacrylamid wurde hergestellt aus Acetoncyanhydrin und Schwefelsäure mit einem Gesamtwassergehalt von 5,62 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 5 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 5 zusammengefasst sind. Die sich daraus ergebende Ausbeute ist ebenfalls in Tabellen 5 und 10 dargestellt.

Die mittlere Ausbeute von 10 repräsentativen Proben, gemessen im stationären Betrieb des Prozesses mittels HPLC, betrug unter den genannten Bedingungen 96,7 %.

| **Tabelle 5: Ergebnisse erfindungsgem. Beispiel 5** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäure amid | Methacrylam id + Methacrylsä ure |
| 1 | Tag 1; 10:00 | 36,21 | 0,93 | 0,22 | 96,24 |
| 2 | Tag 1; 13:50 | 36,30 | 0,94 | 0,26 | 96,69 |
| 3 | Tag 2; 07:40 | 36,32 | 0,96 | 0,26 | 96,81 |
| 4 | Tag 2; 11:23 | 36,34 | 0,92 | 0,30 | 96,84 |
| 5 | Tag 3; 08:05 | 36,36 | 0,92 | 0,31 | 96,91 |
| 6 | Tag 3; 11:05 | 36,40 | 0,92 | 0,29 | 96,90 |
| 7 | Tag 4; 09:34 | 36,27 | 0,94 | 0,28 | 96,65 |
| 8 | Tag 4; 13:10 | 36,35 | 0,95 | 0,26 | 96,71 |
| 9 | Tag 5; 08:40 | 36,30 | 0,95 | 0,25 | 96,63 |
| 10 | Tag 5; 11:18 | 36,37 | 0,93 | 0,22 | 96,81 |
| **Mittelwert** | | | | | **96,7** |
| | | | | | Schwankung sbreite der Ausbeute 0,7% |

### Vergleichsbeispiel 6:

Methacrylamid wurde hergestellt aus Acetoncyanhydrin und Schwefelsäure mit einem Gesamtwassergehalt von 5,62 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 10 dargestellt. Soweit nicht anders angegeben, wurde das Verfahren unter identischen Prozessbedingungen wie im erfindungsgemäßen Beispiel 1 durchgeführt.

Abweichend zu den vorangegangenen Beispielen, wurden die Amidierungs-Reaktoren I (D) und (F) bei 80°C statt bei 98°C betrieben. Diese deutlich niedrigere Amidierungstemperatur in der zweiten Reaktionsstufe, führte zu einer drastischen Viskositätserhöhung des Reaktionsgemischs (11, 13), welche umgehend die für den Wärmeaustausch notwendige Zirkulation des Reaktionsmediums im ersten Reaktor I (D) bzw. im zweiten Reaktor I (F) drastisch reduzierte. Diese Betriebsweise führte daraufhin zu partieller Unterkühlung des Reaktionsmediums in Teilbereichen der Kühlwasser-betriebenen Wärmetauschern des ersten Reaktors I (D) bzw. des zweiten Reaktors I (F) und führte schließlich zu Fällungsreaktionen. Hierdurch verblockte die Anlage und musste abgestellt werden.

Aus diesem Grund konnten keine Analysendaten bezüglich der Konzentrationen im stationären Betrieb erhoben werden.

### Vergleichsbeispiel 7:

Methacrylamid wurde hergestellt aus Acetoncyanhydrin und Schwefelsäure mit einem Gesamtwassergehalt von 20,66 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 6 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 6 zusammengefasst sind. Die sich daraus ergebende Ausbeute ist ebenfalls in Tabellen 6 und 10 dargestellt.

Die mittlere Ausbeute von 10 repräsentativen Proben, gemessen im stationären Betrieb des Prozesses mittels HPLC, betrug unter den genannten Bedingungen 89,1 %.

Es lag eine sehr wässrige Fahrweise im Zulauf (8a, 10) zur Amidierung vor und selbst durch eine Anpassung der Konvertierungsbedingungen (vgl. Tabelle 10) konnte nur eine sehr geringe Ausbeute erzielt werden. Durch den hohen Wasseranteil in den Amidierungsreaktoren (D, F) war es nicht möglich, das in der Amidierung (D, F) zwangsläufig gebildete HIBA zu MASA umzusetzen, ohne dabei bereits vorhandenes MASA bereits zu zersetzen.

| **Tabelle 6: Ergebnisse Vergleichsbeispiel 7** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäure amid | Methacrylam id + Methacrylsä ure |
| 1 | Tag 1; 08:05 | 32,57 | 1,71 | 2,30 | 89,92 |
| 2 | Tag 1; 10:05 | 32,23 | 1,62 | 2,37 | 88,98 |
| 3 | Tag 2; 07:45 | 32,39 | 1,63 | 2,39 | 89,42 |
| 4 | Tag 2; 11:45 | 32,28 | 0,89 | 2,21 | 89,12 |
| 5 | Tag 3; 08:15 | 31,92 | 1,77 | 2,80 | 88,13 |
| 6 | Tag 3; 11:05 | 32,19 | 1,58 | 2,12 | 88,87 |
| 7 | Tag 4; 08:32 | 31,90 | 1,62 | 2,74 | 88,07 |
| 8 | Tag 4; 11:19 | 32,61 | 1,59 | 2,40 | 90,03 |
| 9 | Tag 5; 08:21 | 32,54 | 1,62 | 2,30 | 89,84 |
| 10 | Tag 5; 11:19 | 32,10 | 1,80 | 2,22 | 88,62 |
| **Mittelwert** | | | | | **89,1** |
| | | | | | Schwankung sbreite der Ausbeute 2 % |

### Vergleichsbeispiel 8:

Methacrylamid wurde hergestellt aus Acetoncyanhydrin und Schwefelsäure mit einem Gesamtwassergehalt von 3,66 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 7 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 7 zusammengefasst sind. Die sich daraus ergebende Ausbeute ist ebenfalls in Tabellen 7 und 10 dargestellt.

Die mittlere Ausbeute von 10 repräsentativen Proben, gemessen im stationären Betrieb des Prozesses mittels HPLC, betrug unter den genannten Bedingungen 89,9 %.

Der Amidierung wurde im Schwefelsäure-Zulauf (10) Oleum (100,5% H₂SO₄, 0,5% freies SO₃) zugeführt, und selbst durch Anpassung der Konvertierungsbedingungen (vgl. Tabelle 10) konnte nur eine geringe Ausbeute erzielt werden. Überraschenderweise war es in Kombination mit Oleum im Schwefelsäure-Zulauf (10), also mit einer Zufuhr von Wasser in die Amidierung (D, F) lediglich über den ACH-Zulauf (8a), nicht möglich, hohe Ausbeuten zu erzielen. Ein hoher Wasseranteil im ACH-Zulauf (8a), welcher beispielsweise in den erfindungsgemäßen Beispielen 1 bis 4 zu einer guten Ausbeute führte, erwies sich in Kombination mit Oleum im Schwefelsäure-Zulauf (10) als nachteilig.

| **Tabelle 7: Ergebnisse Vergleichsbeispiel 8** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäure amid | Methacrylami d + Methacrylsäur e |
| 1 | Tag 1; 07:00 | 34,10 | 0,29 | 0,23 | 90,49 |
| 2 | Tag 1; 10:08 | 33,91 | 0,14 | 0,15 | 89,99 |
| 3 | Tag 2; 08:40 | 33,73 | 0,15 | 0,15 | 89,51 |
| 4 | Tag 2; 11:25 | 33,52 | 0,22 | 0,20 | 88,95 |
| 5 | Tag 3; 07:23 | 33,49 | 0,26 | 0,21 | 88,87 |
| 6 | Tag 3; 11:02 | 33,22 | 0,25 | 0,22 | 88,15 |
| 7 | Tag 4; 07:49 | 34,06 | 0,26 | 0,25 | 90,38 |
| 8 | Tag 4; 11:10 | 34,08 | 0,28 | 0,29 | 90,44 |
| 9 | Tag 5; 08:13 | 34,29 | 0,24 | 0,22 | 90,99 |
| 10 | Tag 5; 11:17 | 34,26 | 0,24 | 0,23 | 90,91 |
| **Mittelwert** | | | | | **89,9** |
| | | | | | Schwankungs breite der Ausbeute 2,4 % |

### Vergleichsbeispiel 9:

Methacrylamid wurde hergestellt aus Acetoncyanhydrin und Schwefelsäure mit einem Gesamtwassergehalt von 0,47 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabelle 8 bzw. 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 8 zusammengefasst sind. Die sich daraus ergebende Ausbeute ist ebenfalls in Tabellen 8 und 10 dargestellt.

Die mittlere Ausbeute von 10 repräsentativen Proben, gemessen im stationären Betrieb des Prozesses mittels HPLC, betrug unter den genannten Bedingungen 90,9 %.

Es lag eine wasserarme Fahrweise im ACH-Zulauf (8a, 10) zur Amidierung vor und selbst durch Anpassung der Konvertierungsbedingungen (vgl. Tabelle 10) konnte nur eine geringe Ausbeute erzielt werden. Das fehlende Wasser im Schwefelsäure-Zulauf (10) konnte durch den Wassergehalt im ACH-Zulauf (8a) nicht ausgeglichen werden.

| **Tabelle 8: Ergebnisse Vergleichsbeispiel 9** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersä ureamid | Methacrylamid + Methacrylsäure |
| 1 | Tag 1; 07:22 | 34,85 | 0,04 | 0,02 | 91,60 |
| 2 | Tag 1; 10:56 | 34,60 | 0,05 | 0,02 | 90,94 |
| 3 | Tag 2; 08:07 | 34,73 | 0,04 | 0,02 | 91,28 |
| 4 | Tag 2; 10:05 | 34,64 | 0,03 | 0,06 | 91,05 |
| 5 | Tag 3; 07:03 | 34,32 | 0,00 | 0,04 | 90,21 |
| 6 | Tag 3; 11:16 | 34,78 | 0,00 | 0,01 | 91,42 |
| 7 | Tag 4; 09:49 | 34,77 | 0,03 | 0,00 | 91,39 |
| 8 | Tag 4; 12:10 | 34,68 | 0,05 | 0,02 | 91,15 |
| 9 | Tag 5; 08:41 | 34,29 | 0,04 | 0,03 | 90,13 |
| 10 | Tag 5; 11:53 | 34,14 | 0,04 | 0,03 | 89,73 |
| **Mittelwert** | | | | | **90,9** |
| | | | | | Schwankungsbre ite der Ausbeute 1,9 % |

### Vergleichsbeispiel 10:

Methacrylamid wurde hergestellt aus Acetoncyanhydrin und Schwefelsäure mit einem Gesamtwassergehalt von 0,05 mol-% im Zulauf (8a, 10) der Amidierung in der zweiten Reaktionsstufe (D, F), bezogen auf die Gesamtmenge an ACH, die der zweiten Reaktionsstufe zugeführt wurde (8a). Die einzelnen Wassergehalte der Einsatzstoffe ACH (8a bzw. 8b und 8c) und Schwefelsäure (10) sind in Tabelle 10 ausgewiesen.

Die Prozessbedingungen mit variiertem Wassergehalt hinsichtlich der Einsatzstoffe ACH (8a) und Schwefelsäure (10) der zweiten Reaktionsstufe (D, F) sind in Tabellen 9 und 10 dargestellt. Soweit nicht anders angegeben, wurde das Verfahren unter identischen Prozessbedingungen wie im erfindungsgemäßen Beispiel 1 durchgeführt.

Durch die Variation des Wassergehalts im Zulauf (8a, 10) zur Amidierung (D, F) ergaben sich Reaktionsbedingungen, deren Auswirkung auf die Konzentration im dritten Reaktionsgemisch (14) nach dem zweiten Konverter (G) in Tabelle 9 zusammengefasst sind. Die sich daraus ergebende Ausbeute ist ebenfalls in Tabellen 9 und 10 dargestellt.

Die mittlere Ausbeute von drei repräsentativen Proben, gemessen im stationären Betrieb des Prozesses mittels HPLC, betrug unter den genannten Bedingungen 89,5 %.

Es lag eine wasserarme Fahrweise im ACH-Zulauf (8a, 10) zur Amidierung vor, und selbst durch Anpassung der Konvertierungsbedingungen (vgl. Tabelle 10) konnte nur eine geringe Ausbeute erzielt werden. Das fehlende Wasser im Schwefelsäure-Zulauf (10) konnte durch den Wassergehalt im ACH-Zulauf (8a) nicht ausgeglichen werden. Durch den im Vergleich zum Vergleichsbeispiel 9 nochmals verringerten Wassergehalt im ACH-Zulauf (8a) wurde die Ausbeute nochmals signifikant erniedrigt.

| **Tabelle Ergeb Vergl**e **9: nisse ichsbeispiel 10** | | **Konzentration im Produktstrom [Gew.-%]** | | | **Ausbeute [%]** |
|---|---|---|---|---|---|
| Probe | Zeit | Methacrylamid | Methacrylsäure | Hydroxyisobuttersäurea mid | Methacryla mid + Methacryls äure |
| 2 | Tag 1; 10:32 | 34,60 | 0,03 | 0,02 | 90,94 |
| 3 | Tag 2; 08:11 | 34,53 | 0,04 | 0,02 | 91,28 |
| 4 | Tag 2; 10:24 | 34,64 | 0,03 | 0,04 | 91,05 |
| **Mittel wert** | | | | | **89,5** |

### Zusammenfassung der Beispiele:

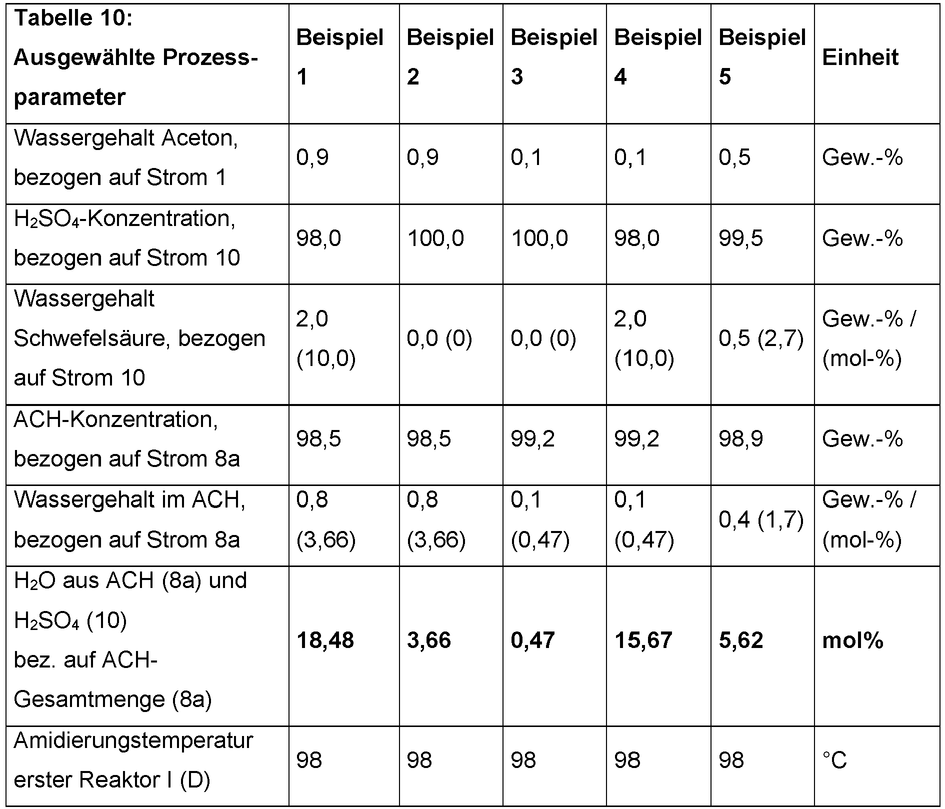

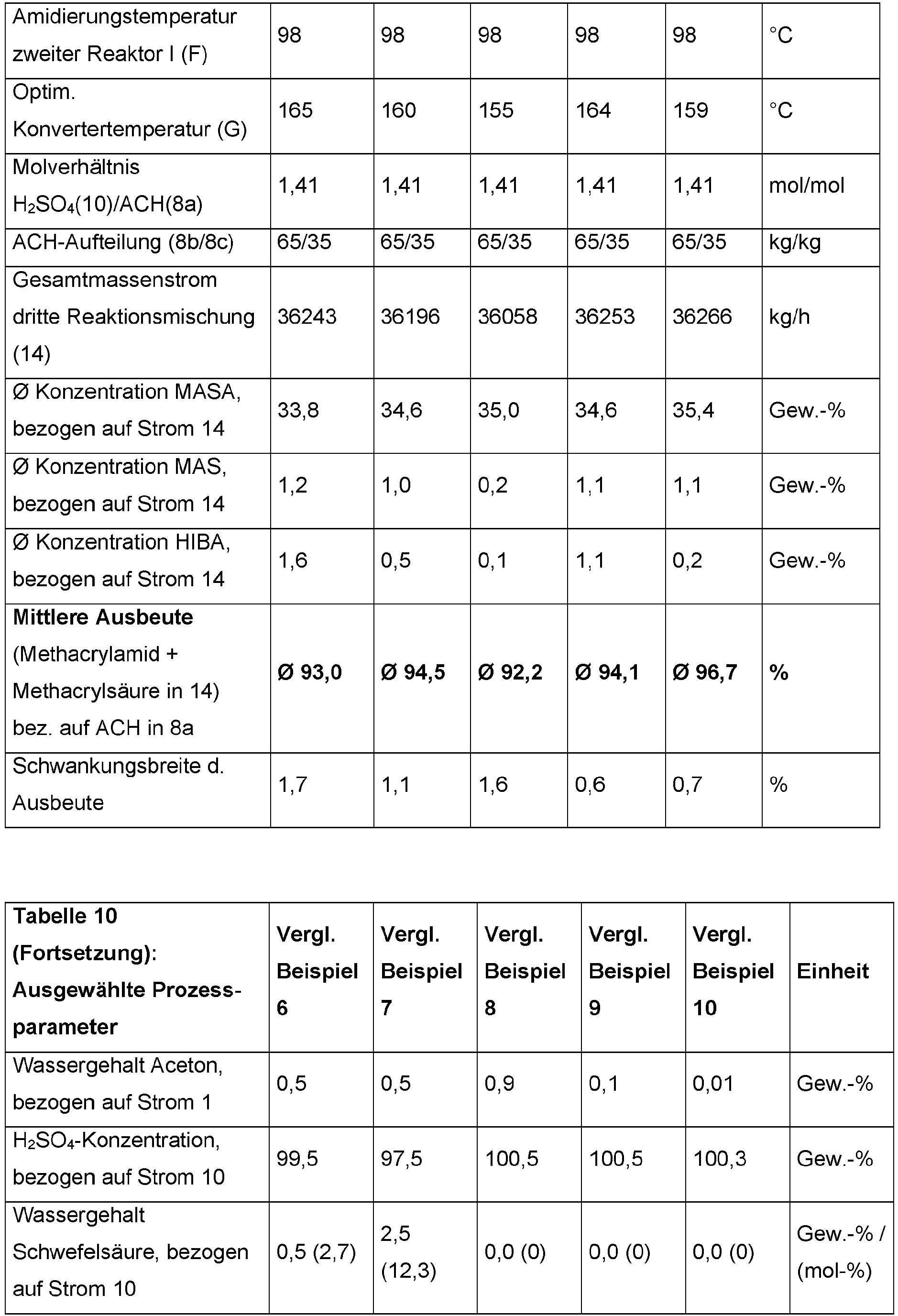

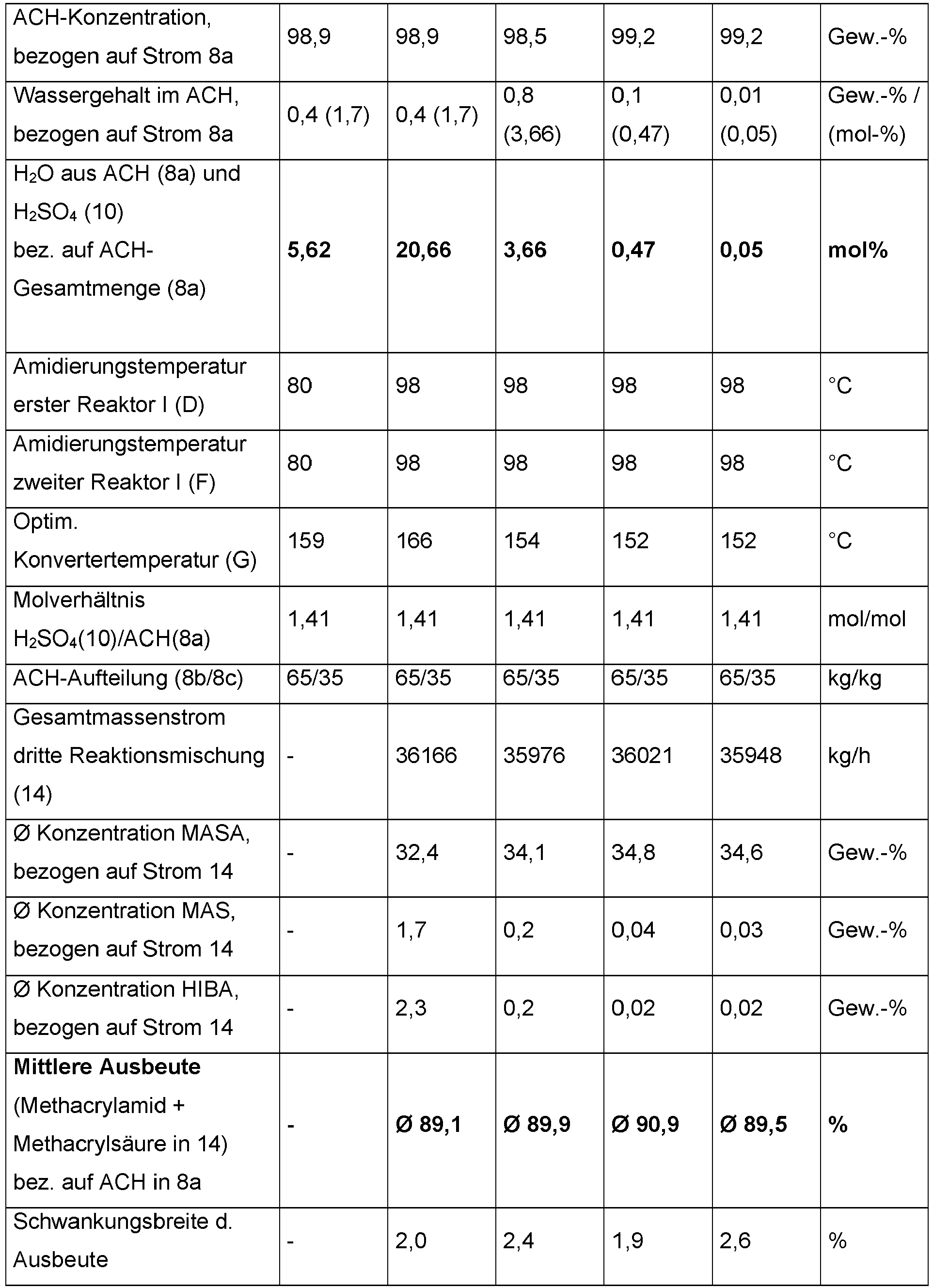

Ein Vergleich der erfindungsgemäßen Beispiele 1 bis 5 hebt hervor, dass ein erfindungsgemäßer Wassergehalt in der Amidierung (D, F, 8a, 10), der sowohl vom Wassergehalt des in die erste Reaktionsstufe (A, B) zugeführten Aceton-Reaktanten (1) als auch vom Wassergehalt der in der Amidierung (D) eingesetzten Schwefelsäure (10) bestimmt ist, essenziell für hohe und stabile Ausbeuten ist. Gemäß Tabelle 10 wurden mit einer Gesamtmenge an Wasser zwischen 0,5 und 18,5 mol-%, bezogen auf eine Gesamtmenge an ACH, die der zweiten Reaktionsstufe zugeführt wird, die höchsten Ausbeuten erzielt, sofern kein Oleum an Stelle von Schwefelsäure eingesetzt wurde (s. Vergleichsbeispiele 8, 9 und 10), welches die Ausbeuten weiter verschlechterte. Weiterhin war die Streubreite der gemessenen Ausbeute in einem Bereich von 6 bis 16 mol-% Gesamtmenge an Wasser, bezogen auf die Gesamtmenge an ACH, die der zweiten Reaktionsstufe zugeführt wurde, am geringsten und somit der gesamte Prozess am stabilsten.

Es zeigte sich ebenfalls, dass neben der zur Erreichung der maximalen Ausbeute führenden Gesamtwassermenge insbesondere die Konzentration der zugeführten Schwefelsäure (10) relevant ist (vgl. Beispiele 2 und 5 sowie 2 und 8).

Zusätzlich stellte sich heraus, dass bei Überschreitung einer Gesamtwassermenge in der Amidierung von 16 mol-%, bezogen auf eine Gesamtmenge an ACH, die der zweiten Reaktionsstufe zugeführt wurde, eine deutliche Zunahme der Komponenten MAS und HIBA in der dritten Reaktionsmischung (14) zu erkennen ist, was unter anderem Ausbeuteverlust mit sich brachte. Zwar ist es möglich, durch Einstellung einer höheren Konvertierungstemperatur (E, G) den Anteil an HIBA, unter Umsetzung zu MASA, zu reduzieren, jedoch sind diesem Prozess Grenzen gesetzt. Beim Vergleich der Beispiele 4, 1 und 7, welche einen steigenden Gesamtwasseranteil von 5,6 auf 18,5 und auf 20,6 mol-% repräsentieren, wurden höhere Konvertierungstemperaturen verwendet, um den zunehmend stärker ansteigenden Anteil an HIBA zu konvertieren. Dies gelang ab mehr als 16 mol-% Gesamtwasseranteil nicht mehr in vollem Umfang (vgl. Beispiel 1 und 4) und führte letztendlich zum Ausbeuteverlust.

Ein Vergleich der Beispiele 2 und 8 knüpft an diesen Effekt an. Der Betrieb mit 3,66 mol-% Gesamtwasser, welcher in beiden Fällen ausschließlich mit dem ACH (8a) zugeführt wurde, führte hierzu unterschiedlichen Ausbeuten. Im Falle der 100%-igen Schwefelsäure (Beispiel 2) ist die erreichte Ausbeute bereits auf einem guten Niveau, während der Einsatz von Oleum (10) in Beispiel 8 zu deutlichen Ausbeuteeinbußen führte. Wird in diesem Zusammenhang der Anteil HIBA sowie MAS in der dritten Reaktionsmischung (14) betrachtet, ist zu erkennen, dass im Falle von 100%-iger Schwefelsäure (Beispiel 2) bei 160 °C Konvertierungstemperatur ein moderater Anteil HIBA und MAS bei guter Ausbeute erzeugt wurde. Obwohl diese Komponenten bei der Oleumfahrweise (100,5 % in Beispiel 8) und einer Konvertierungstemperatur von 152 °C deutlich reduziert auftraten, wurde dennoch eine schlechtere Gesamtausbeute gefunden. Werden die Produktmasseströme (14) der Beispiele 2 und 8 verglichen, wird deutlich, dass der Ausbeuteverlust durch Abgasverluste (reduzierter Produktstrom) zustande kam und damit nicht allein die Bildung von HIBA für den Ausbeuteverlust verantwortlich war. Der Einsatz von Oleum (10) bei hohem Wassergehalt im ACH-Zulauf (8a) führte zu stärkeren Ausbeuteeinbußen, als es bei 100%-iger Schwefelsäure (10) der Fall war.

## Patentansprüche

1. Verfahren zur Herstellung von Methacrylsäure und/oder Alkylmethacrylat, bevorzugt Methylmethacrylat, umfassend:
a. Umsetzung von Aceton und Blausäure in Gegenwart eines basischen Katalysators in einer ersten Reaktionsstufe (Synthese von ACH), wobei eine erste Reaktionsmischung enthaltend Acetoncyanhydrin (ACH) erhalten wird;
b. Aufarbeitung der ersten Reaktionsmischung, enthaltend Acetoncyanhydrin (ACH);
c. Umsetzung von Acetoncyanhydrin und Schwefelsäure in einem oder mehreren Reaktoren I in einer zweiten Reaktionsstufe (Amidierung) bei einer Amidierungstemperatur im Bereich von 85°C bis 130°, wobei eine zweite Reaktionsmischung, enthaltend Sulfoxyisobuttersäureamid und Methacrylamid, erhalten wird;
d. Konvertieren der zweiten Reaktionsmischung, umfassend Erhitzen auf eine Konvertierungstemperatur im Bereich von 130°C bis 200°C, in einem oder mehreren Reaktoren II in einer dritten Reaktionsstufe (Konvertierung), wobei eine dritte Reaktionsmischung, enthaltend überwiegend Methacrylamid (MASA) und Schwefelsäure, erhalten wird;
e. Umsetzung der dritten Reaktionsmischung mit Wasser und optional Alkohol, bevorzugt Wasser und optional Methanol, in einem oder mehreren Reaktoren III in einer vierten Reaktionsstufe (Hydrolyse oder Veresterung), wobei eine vierte Reaktionsmischung, enthaltend Methacrylsäure und/oder Alkylmethacrylat, bevorzugt Methylmethacrylat, erhalten wird;
f. gegebenenfalls Abtrennung von Alkylmethacrylat aus der vierten Reaktionsmischung erhalten aus der vierten Reaktionsstufe;
wobei die in der zweiten Reaktionsstufe eingesetzte Schwefelsäure eine Konzentration im Bereich von 98,0 Gew.-% bis 100,0 Gew.-% aufweist,
wobei in der zweiten Reaktionsstufe eine Rein-ACH-Mischung eingesetzt wird, die einen Wassergehalt in einem Bereich von 0,1 mol-% bis 10 mol-%, insbesondere 0,4 mol-% bis 5 mol-% aufweist, bezogen auf in der Rein-ACH-Mischung enthaltenes ACH,
und wobei in der zweiten Reaktionsstufe eine Gesamtmenge an Wasser in einem Bereich von 0,1 mol-% bis 20 mol-%, insbesondere 0,4 mol-% bis 10 mol-% eingesetzt wird, bezogen auf in der Rein-ACH-Mischung enthaltenes ACH.

2. Verfahren nach Anspruch 1, **dadurch gekennzeichnet, dass** die Aufarbeitung der ersten Reaktionsmischung in Schritt b. einen Destillationsschritt umfasst, wobei Acetoncyanhydrin zumindest teilweise von Verunreinigungen und / oder Nebenprodukten, die niedrigsiedender sind als Acetoncyanhydrin, abgetrennt wird.

3. Verfahren nach einem der Ansprüche 1 bis 2, **dadurch gekennzeichnet, dass** in der ersten Reaktionsstufe ein Aceton-Reaktant eingesetzt wird, der 0,1 Gew.-% bis 1 Gew.-%, insbesondere 0,1 Gew.-% bis 0,5 Gew.-%, Wasser enthält, bezogen auf den gesamten Aceton-Reaktanten.

4. Verfahren nach einem der Ansprüche 1 bis 3, **dadurch gekennzeichnet, dass** in der ersten Reaktionsstufe ein Blausäure-Reaktant eingesetzt wird, der 0,01 Gew.-% bis 0,1 Gew.-% Wasser enthält, bezogen auf den gesamten Blausäure-Reaktanten.

5. Verfahren nach einem der Ansprüche 1 bis 4, **dadurch gekennzeichnet, dass** die Gesamtmenge an ACH, die der zweiten Reaktionsstufe zugeführt wird, mit der Rein-ACH-Mischung zugeführt wird.

6. Verfahren nach einem der Ansprüche 1 bis 5, **dadurch gekennzeichnet, dass** Schwefelsäure und Acetoncyanhydrin (ACH) in der zweiten Reaktionsstufe in einem molaren Verhältnis von Schwefelsäure zu ACH im Bereich von 1,3 bis 1,8; bevorzugt von 1,4 bis 1,6, eingesetzt werden, bezogen auf die Gesamtmenge an ACH, die der zweiten Reaktionsstufe zugeführt wird.

7. Verfahren nach einem der Ansprüche 1 bis 6, **dadurch gekennzeichnet, dass** die dritte Reaktionsmischung, enthaltend Methacrylamid (MASA), kleiner oder gleich 3 Gew.-% Methacrylsäure (MAS), kleiner oder gleich 2 Gew.-% alpha-Hydroxyisobuttersäureamid (HIBA) und kleiner oder gleich 0,3 Gew.-% Methacrylnitril (MAN), jeweils bezogen auf die gesamte dritte Reaktionsmischung, enthält.

8. Verfahren nach einem der Ansprüche 1 bis 7, **dadurch gekennzeichnet, dass** die dritte Reaktionsmischung 30 Gew.-% bis 40 Gew.-% Methacrylamid (MASA), bezogen auf die gesamte dritte Reaktionsmischung, enthält.

9. Verfahren nach einem der Ansprüche 1 bis 8, **dadurch gekennzeichnet, dass** die zweite Reaktionsstufe die Umsetzung von Acetoncyanhydrin (ACH) und Schwefelsäure in mindestens zwei getrennten Reaktionszonen umfasst.

10. Verfahren nach einem der Ansprüche 1 bis 9, **dadurch gekennzeichnet, dass** die zweite Reaktionsstufe die Umsetzung von Acetoncyanhydrin (ACH) und Schwefelsäure in mindestens zwei getrennten Reaktoren I umfasst, wobei Schwefelsäure und Acetoncyanhydrin (ACH) in einem ersten Reaktor I in einem molaren Verhältnis von Schwefelsäure zu ACH im Bereich von 1,6 bis 3,0 eingesetzt werden, bezogen auf im ersten Reaktor I eingesetztes ACH, und wobei Schwefelsäure und Acetoncyanhydrin (ACH) in einem zweiten Reaktor I in einem molaren Verhältnis von Schwefelsäure zu ACH im Bereich von 1,2 bis 2,0 eingesetzt werden, bezogen auf eine Gesamtmenge an ACH, die der zweiten Reaktionsstufe zugeführt wird.

11. Verfahren nach einem der Ansprüche 1 bis 10, **dadurch gekennzeichnet, dass** die vierte Reaktionsmischung, enthaltend Alkylmethacrylat, die in der vierten Reaktionsstufe erhalten wird, in weiteren Schritten umfassend mindestens einen Destillationsschritt und/oder mindestens einen Extraktionsschritt aufgearbeitet wird.

12. Verfahren nach einem der Ansprüche 1 bis 11, **dadurch gekennzeichnet, dass** die vierte Reaktionsmischung, enthaltend Alkylmethacrylat, die in der vierten Reaktionsstufe erhalten wird, in gasförmiger Form in einen Destillationsschritt geführt wird, wobei eine Kopf-Fraktion, enthaltend Alkylmethacrylat, Wasser und Alkohol, und eine Sumpf-Fraktion enthaltend schwerer siedende Komponenten, erhalten werden, und wobei die Sumpf-Fraktion vollständig oder teilweise in die vierte Reaktionsstufe zurückgeführt wird.

13. Verfahren nach Anspruch 12, **dadurch gekennzeichnet, dass** die Kopf-Fraktion, enthaltend Alkylmethacrylat, Wasser und Alkohol, in einem Phasentrennungsschritt in eine organische Phase, enthaltend den überwiegenden Teil des Alkylmethacrylats, und in eine wässrige Phase, enthaltend Alkohol und weitere wasserlösliche Verbindungen, getrennt wird, und wobei die wässrige Phase vollständig oder teilweise in die dritte Reaktionsstufe zurückgeführt wird, und die organische Phase, enthaltend den überwiegenden Teil des Alkylmethacrylats, einer Extraktion unter Verwendung von Wasser als Extraktionsmittel unterzogen wird, wobei die wässrige Phase dieser Extraktion in die dritte Reaktionsstufe zurückgeführt wird.

## Claims

1. Process for preparing methacrylic acid and/or alkyl methacrylate, preferably methyl methacrylate, comprising:
a. reacting acetone and hydrogen cyanide in the presence of a basic catalyst in a first reaction stage (synthesis of ACH) to obtain a first reaction mixture comprising acetone cyanohydrin (ACH);
b. working up the first reaction mixture comprising acetone cyanohydrin (ACH);
c. reacting acetone cyanohydrin and sulfuric acid in one or more reactors I in a second reaction stage (amidation) at an amidation temperature in the range from 85°C to 130°C to obtain a second reaction mixture comprising sulfoxyisobutyramide and methacrylamide;
d. converting the second reaction mixture, comprising heating to a conversion temperature in the range from 130°C to 200°C, in one or more reactors II in a third reaction stage (conversion) to obtain a third reaction mixture comprising predominantly methacrylamide (MAA) and sulfuric acid;
e. reacting the third reaction mixture with water and optionally alcohol, preferably water and optionally methanol, in one or more reactors III in a fourth reaction stage (hydrolysis or esterification) to obtain a fourth reaction mixture comprising methacrylic acid and/or alkyl methacrylate, preferably methyl methacrylate;
f. optionally separating alkyl methacrylate from the fourth reaction mixture obtained from the fourth reaction stage;
wherein the sulfuric acid used in the second reaction stage has a concentration in the range from 98.0% by weight to 100.0% by weight;
wherein a pure ACH mixture which is used in the second reaction stage has a water content within a range from 0.1 mol% to 10 mol%, especially 0.4 mol% to 5 mol%, based on ACH present in the pure ACH mixture,
and wherein a total amount of water used in the second reaction stage is within a range from 0.1 mol% to 20 mol%, especially 0.4 mol% to 10 mol%, based on ACH present in the pure ACH mixture.

2. Process according to Claim 1, **characterized in that** the workup of the first reaction mixture in step b. comprises a distillation step, wherein acetone cyanohydrin is separated at least partly from impurities and/or by-products that are lowerboiling than acetone cyanohydrin.

3. Process according to either of Claims 1 and 2, **characterized in that** an acetone reactant which is used in the first reaction stage contains 0.1% by weight to 1% by weight, especially 0.1% by weight to 0.5% by weight, of water, based on the overall acetone reactants.

4. Process according to any of Claims 1 to 3, **characterized in that** a hydrogen cyanide reactant which is used in the first reaction stage contains 0.01% by weight to 0.1% by weight of water, based on the overall hydrogen cyanide reactants.

5. Process according to any of Claims 1 to 4, **characterized in that** the total amount of ACH fed to the second reaction stage is fed in with the pure ACH mixture.

6. Process according to any of Claims 1 to 5, **characterized in that** sulfuric acid and acetone cyanohydrin (ACH) are used in the second reaction stage in a molar ratio of sulfuric acid to ACH in the range from 1.3 to 1.8, preferably from 1.4 to 1.6, based on the total amount of ACH fed to the second reaction stage.

7. Process according to any of Claims 1 to 6, **characterized in that** the third reaction mixture comprising methacrylamide (MAA) contains not more than 3% by weight of methacrylic acid (MA), not more than 2% by weight of alpha-hydroxyisobutyramide (HIBAm) and not more than 0.3% by weight of methacrylonitrile (MAN), based in each case on the overall third reaction mixture.

8. Process according to any of Claims 1 to 7, **characterized in that** the third reaction mixture contains 30% by weight to 40% by weight of methacrylamide (MAA), based on the overall third reaction mixture.

9. Process according to any of Claims 1 to 8, **characterized in that** the second reaction stage comprises the conversion of acetone cyanohydrin (ACH) and sulfuric acid in at least two separate reaction zones.

10. Process according to any of Claims 1 to 9, **characterized in that** the second reaction stage comprises the conversion of acetone cyanohydrin (ACH) and sulfuric acid in at least two separate reactors I, wherein sulfuric acid and acetone cyanohydrin (ACH) are used in a first reactor I in a molar ratio of sulfuric acid to ACH in the range from 1.6 to 3.0, based on ACH used in the first reactor I, and wherein sulfuric acid and acetone cyanohydrin (ACH) are used in a second reactor I in a molar ratio of sulfuric acid to ACH in the range from 1.2 to 2.0, based on a total amount of ACH fed into the second reaction stage.

11. Process according to any of Claims 1 to 10, **characterized in that** the fourth reaction mixture comprising alkyl methacrylate which is obtained in the fourth reaction stage is worked up in further steps comprising at least one distillation step and/or at least one extraction step.

12. Process according to any of Claims 1 to 11, **characterized in that** the fourth reaction mixture comprising alkyl methacrylate which is obtained in the fourth reaction stage is guided in gaseous form into a distillation step, wherein a tops fraction comprising alkyl methacrylate, water and alcohol, and a bottoms fraction comprising higher-boiling components are obtained, and wherein the bottoms fraction is recycled fully or partly into the fourth reaction stage.

13. Process according to Claim 12, **characterized in that** the tops fraction comprising alkyl methacrylate, water and alcohol is separated in a phase separation step into an organic phase comprising the predominant portion of the alkyl methacrylate and into an aqueous phase comprising alcohol and further water-soluble compounds, and wherein the aqueous phase is recycled fully or partly into the third reaction stage, and the organic phase comprising the predominant portion of the alkyl methacrylate is subjected to an extraction using water as extractant, wherein the aqueous phase from this extraction is recycled into the third reaction stage.

## Revendications

1. Procédé de préparation de l'acide méthacrylique et/ou d'un méthacrylate d'alkyle, préférablement de méthacrylate de méthyle, comprenant :
a. transformation d'acétone et de cyanure d'hydrogène en présence d'un catalyseur basique dans un premier stade de réaction (synthèse d'ACH), un premier mélange réactionnel étant obtenu contenant de la cyanhydrine d'acétone (ACH) ;
b. traitement du premier mélange réactionnel, contenant de la cyanhydrine d'acétone (ACH) ;
c. transformation de la cyanhydrine d'acétone et d'acide sulfurique dans un ou plusieurs réacteurs I dans un deuxième stade de réaction (amidation) à une température d'amidation dans la plage de 85 °C à 130°, un deuxième mélange réactionnel étant obtenu contenant de l'amide d'acide sulfoxyisobutyrique et du méthacrylamide ;
d. conversion du deuxième mélange réactionnel, comprenant un chauffage à une température de conversion dans la plage de 130 °C à 200 °C, dans un ou plusieurs réacteurs II dans un troisième stade de réaction (conversion), un troisième mélange réactionnel étant obtenu contenant majoritairement du méthacrylamide (MASA) et de l'acide sulfurique,
e. transformation du troisième mélange réactionnel avec de l'eau et éventuellement un alcool, préférablement de l'eau et éventuellement du méthanol, dans un ou plusieurs réacteurs III dans un quatrième stade de réaction (hydrolyse ou estérification), un quatrième mélange réactionnel étant obtenu contenant de l'acide méthacrylique et/ou un méthacrylate d'alkyle, préférablement du méthacrylate de méthyle ;
f. éventuellement séparation du méthacrylate d'alkyle du quatrième mélange réactionnel obtenu du quatrième stade de réaction ;
l'acide sulfurique utilisé dans le deuxième stade de réaction présentant une concentration dans la plage de 98,0 % en poids à 100,0 % en poids,
dans le deuxième stade de réaction, un mélange d'ACH pur étant utilisé, qui présente une teneur en eau dans une plage de 0,1 % en moles à 10 % en moles, en particulier de 0,4 % en moles à 5 % en moles, par rapport à l'ACH contenue dans le mélange d'ACH pur, et dans le deuxième stade de réaction, une quantité totale d'eau dans une plage de 0,1 % en moles à 20 % en moles, en particulier de 0,4 % en moles à 10 % en moles, étant utilisée, par rapport à l'ACH contenue dans le mélange d'ACH pur.

2. Procédé selon la revendication 1, **caractérisé en ce que** le traitement du premier mélange réactionnel dans l'étape b. comprend une étape de distillation, la cyanhydrine d'acétone étant séparée au moins partiellement d'impuretés et/ou de sous-produits à point d'ébullition plus bas que celui de la cyanhydrine d'acétone.

3. Procédé selon l'une quelconque des revendications 1 à 2, **caractérisé en ce que** dans le premier stade de réaction, un réactant d'acétone est utilisé qui contient 0,1 % en poids à 1 % en poids, en particulier 0,1 % en poids à 0,5 % en poids, d'eau, par rapport aux réactants d'acétone totaux.

4. Procédé selon l'une quelconque des revendications 1 à 3, **caractérisé en ce que** dans le premier stade de réaction, un réactant de cyanure d'hydrogène est utilisé qui contient 0,01 % en poids à 0,1 % en poids d'eau, par rapport aux réactants de cyanure d'hydrogène totaux.

5. Procédé selon l'une quelconque des revendications 1 à 4, **caractérisé en ce que** la quantité totale d'ACH, qui est introduite au deuxième stade de réaction, est introduite avec le mélange d'ACH pur.

6. Procédé selon l'une quelconque des revendications 1 à 5, **caractérisé en ce que** l'acide sulfurique et la cyanhydrine d'acétone (ACH) dans le deuxième stade de réaction sont utilisés en un rapport molaire d'acide sulfurique sur ACH dans la plage de 1,3 à 1,8 ; préférablement de 1,4 à 1,6, par rapport à la quantité totale d'ACH qui est introduite au deuxième stade de réaction.

7. Procédé selon l'une quelconque des revendications 1 à 6, **caractérisé en ce que** le troisième mélange réactionnel, contenant du méthacrylamide (MASA), contient une quantité inférieure ou égale à 3 % en poids d'acide méthacrylique (MAS), une quantité inférieure ou égale à 2 % en poids d'amide d'acide alpha-hydroxyisobutyrique (HIBA) et une quantité inférieure ou égale à 0,3 % en poids de méthacrylonitrile (MAN), à chaque fois par rapport au troisième mélange réactionnel total.

8. Procédé selon l'une quelconque des revendications 1 à 7, **caractérisé en ce que** le troisième mélange réactionnel contient 30 % en poids à 40 % en poids de méthacrylamide (MASA), par rapport au troisième mélange réactionnel total.

9. Procédé selon l'une quelconque des revendications 1 à 8, **caractérisé en ce que** le deuxième stade de réaction comprend la transformation de cyanhydrine d'acétone (ACH) et d'acide sulfurique dans au moins deux zones de réaction séparées.

10. Procédé selon l'une quelconque des revendications 1 à 9, **caractérisé en ce que** le deuxième stade de réaction comprend la transformation de cyanhydrine d'acétone (ACH) et d'acide sulfurique dans au moins deux réacteurs I séparés, l'acide sulfurique et la cyanhydrine d'acétone (ACH) étant utilisés dans un premier réacteur I en un rapport molaire d'acide sulfurique sur ACH dans la plage de 1,6 à 3,0, par rapport à l'ACH utilisée dans le premier réacteur I, et l'acide sulfurique et la cyanhydrine d'acétone (ACH) étant utilisés dans un deuxième réacteur I en un rapport molaire d'acide sulfurique sur ACH dans la plage de 1,2 à 2,0, par rapport à la quantité totale d'ACH qui est introduite au deuxième stade de réaction.

11. Procédé selon l'une quelconque des revendications 1 à 10, **caractérisé en ce que** le quatrième mélange réactionnel, contenant un méthacrylate d'alkyle, qui est obtenu dans le quatrième stade de réaction, est traité dans des étapes supplémentaires comprenant au moins une étape de distillation et/ou au moins une étape d'extraction.

12. Procédé selon l'une quelconque des revendications 1 à 11, **caractérisé en ce que** le quatrième mélange réactionnel, contenant un méthacrylate d'alkyle, qui est obtenu dans le quatrième stade de réaction, est conduit sous forme gazeuse dans une étape de distillation, une fraction de tête, contenant un méthacrylate d'alkyle, de l'eau et un alcool, et une fraction de fond contenant des composants à point d'ébullition plus élevé, étant obtenues, et la fraction de fond étant renvoyée entièrement ou partiellement dans le quatrième stade de réaction.

13. Procédé selon la revendication 12, **caractérisé en ce que** la fraction de tête, contenant un méthacrylate d'alkyle, de l'eau et un alcool, est séparée dans une étape de séparation de phases, en une phase organique, contenant la partie principale du méthacrylate d'alkyle, et en une phase aqueuse, contenant un alcool et d'autres composés solubles dans l'eau, et la phase aqueuse étant renvoyée entièrement ou partiellement dans le troisième stade de réaction, et la phase organique, contenant la partie principale du méthacrylate alkyle, étant soumise à extraction en utilisant de l'eau en tant qu'agent d'extraction, la phase aqueuse de cette extraction étant renvoyée dans le troisième stade de réaction.
